(19)

Europäisches
Patentamt

European
Patent Office

Office européen
des brevets

(11) **EP 4 653 001 A1**

(12) **EUROPEAN PATENT APPLICATION**

(43) Date of publication:
**26.11.2025 Bulletin 2025/48**

(21) Application number: **24382547.8**

(22) Date of filing: **23.05.2024**

(51) International Patent Classification (IPC):
*A61K 31/7084* (2006.01)    *A61K 48/00* (2006.01)
*A61P 25/00* (2006.01)    *A61P 25/28* (2006.01)
*C12N 15/86* (2006.01)    *C12Q 1/32* (2006.01)
*G01N 33/50* (2006.01)

(52) Cooperative Patent Classification (CPC):
**A61K 31/7084; A61K 48/005; A61K 48/0075;
A61P 25/00; A61P 25/28; C12N 9/0006;
C12N 15/113; C12Q 1/32;** C12N 2750/14143;
G01N 2333/904

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Universidad Autónoma de Madrid
28049 Madrid (ES)**

(72) Inventor: **BALSA MARTÍNEZ, Eduardo
28049 Ciudad Universitaria de Cantoblanco,
Calle
Nicolás Cabrera 1 (ES)**

(74) Representative: **Hoffmann Eitle
Hoffmann Eitle S.L.U.
Paseo de la Castellana 140, 3a planta
Edificio LIMA
28046 Madrid (ES)**

Remarks:
The complete document including Reference
Table(s) and the Sequence Listing(s) can be
downloaded from the EPO website

(54) **TREATMENT, DIAGNOSIS AND/OR PROGNOSIS OF MITOCHONDRIAL DISEASES**

(57)    The present invention refers to a method for the treatment, diagnosis and/or prognosis of mitochondrial diseases (i.e., pathologies associated with mitochondrial dysfunction), preferably for the treatment, diagnosis and/or prognosis of neurodegenerative mitochondrial diseases (i.e., treatment, diagnosis and/or prognosis of neuroinflammation and/or neurodegeneration in the context of those mitochondrial diseases).

**EP 4 653 001 A1**

Processed by Luminess, 75001 PARIS (FR)

## Description

## FIELD OF THE INVENTION

The present invention refers to the medical field. Particularly, the present invention refers to a method for the treatment, diagnosis and/or prognosis of mitochondrial diseases (i.e., pathologies associated with mitochondrial dysfunction), preferably for the treatment, diagnosis and/or prognosis of neurodegenerative mitochondrial diseases (i.e., treatment, diagnosis and/or prognosis of neuroinflammation and/or neurodegeneration in the context of those mitochondrial diseases).

## STATE OF THE ART

Mitochondria are unique and complex organelles that perform essential functions in many aspects of cell biology. Within these organelles resides the oxidative phosphorylation system (OXPHOS), a molecular machinery that enables the oxidation of nutrients in our diet to generate cellular energy. This bioenergetic process requires the transport of electrons to molecular oxygen by the mitochondrial electron transport chain (ETC), which involves four multi-subunit complexes (known as complex I-complex IV) and two mobile electron carriers, ubiquinone (also known as coenzyme Q10) and cytochrome c (cyt c). The respiratory chain generates a transmembrane proton gradient that is harnessed by complex V (also known as ATP synthase) to synthesize ATP. Mutations in mitochondrial or nuclear DNA that compromise the OXPHOS system are the root of a heterogeneous group of genetic inherited disorders referred to as mitochondrial diseases. These conditions present limited therapeutic options, primarily due to the predominantly unknown molecular mechanisms governing these diseases. Evidence indicates that following biochemical failure, adaptive mechanisms are initiated to restore metabolic homeostasis at both the cellular and organismal levels. Those include mitochondrial biogenesis and membrane dynamics that in turn support mitochondrial respiration. Activation of the integrated mitochondrial stress response (ISRmt), that remodels one-carbon (1C) metabolism and antioxidant response. Increased aerobic glycolysis as the primary source of ATP synthesis and rewiring of glutamine metabolism to fulfil an anaplerotic function.

Once considered to be mere sites of ATP generation, it is now well appreciated that mitochondria participate in a wide range of essential functions, such as controlling apoptosis, cell fate or immune response. Because of this multifaceted contribution of mitochondria to key biologic and metabolic pathways, the fundamental pathogenic processes underlying mitochondrial disease have remained elusive, hampering the development of successful therapeutic strategies. In addition, not every cell type or tissue is equally sensitive to the disruption of the ETC and the molecular mechanisms underlying this differential sensitivity are poorly understood.

Besides its canonical role in producing ATP, re-oxidation of NADH to NAD+ is a critical yet unappreciated function of the electron transport chain. For instance, providing electron acceptors for aspartate synthesis is one of the key functions of respiration in proliferating cells. In fact, the expression of hypoxia markers and aspartate levels in primary human tumors are inversely correlated, indicating that tumor hypoxia is sufficient to block ETC and, in turn, aspartate synthesis *in vivo.*

To ensure adequate flow of electrons from cytosolic NADH to an available electron acceptor when mitochondrial respiration is impaired, cells activate alternative pathways that ultimately regenerates cytosolic NAD+. Several metabolic pathways that alleviate reductive stress caused by the accumulation of NADH have been identified. It has been shown that, Glycerol-3-phosphate (Gro3P) shuttle selectively regenerates cytosolic NAD+ under mitochondrial complex I inhibition and boosting Gro3P synthesis promotes shuttle activity to restore proliferation of complex I-impaired cells and ameliorates the detrimental phenotype of Ndufs4-/- mice, and *in vivo* model of mitochondrial complex I deficiency. Additionally, induction of delta-5 and delta-6 desaturases (D5D/D6D), key enzymes responsible for highly unsaturated fatty acid (HUFA) synthesis, regenerate NAD+ from NADH by depositing electrons into Polyunsaturated fatty acids (PUFAs). Although important, these two pathways lag behind the main mechanism to regenerate cytosolic NAD+ which is reduction of pyruvate to lactate by the Lactate dehydrogenase (LDH). In fact, elevation of blood lactate levels is a common feature of mitochondrial disease patients and a reliable biomarker of the disease. This is a direct consequence of increase uptake and utilization of glucose by cells with blockage in the ETC, which is thought to be an adaptive mechanism to generate ATP through glycolysis when mitochondrial respiration is limited. When related to mitochondrial diseases, a present dogma in the field has assumed that ATP depletion and subsequent energetic crisis derived from ETC dysfunction was the main contributor to the pathology. However, recent evidences challenge this perception. OXPHOS-impaired cells cultured in complete medium do not display reduced levels of ATP. In soleus muscle of Ndufs4-/- mice, ATP levels were minimally reduced, and metabolomic analysis in the brain of these mice also failed to detect a drop in ATP. Alternative hypotheses have started to emerge, postulating that dysregulation of intermediary metabolism and disturbed redox homeostasis might as well account for the pathological symptoms.

Glucose entering the cell can be diverted to feed the pentose phosphate pathway, where its oxidative branch is a major source of reducing power in the form of NADPH. Nevertheless, the interdependence of glycolysis and PPP (pentose phosphate pathways) in the context of mitochondrial dysfunction has been largely unexplored and our understanding about the metabolic and molecular mechanisms that conserve NADPH and redox homeostasis in OXPHOS deficient cells are scarce.

Since, as explained above, mitochondrial diseases present limited therapeutic options, primarily due to the predominantly unknown molecular mechanisms governing these diseases, there is an unmet medical need of finding reliable strategies aimed at treating, diagnosing and/or prognosing mitochondrial diseases, preferably neurodegenerative mitochondrial diseases.

The present invention is focused on solving this problem and an innovative method for the treatment, diagnosis and/or prognosis of mitochondrial diseases, preferably neurodegenerative mitochondrial diseases, is herein provided.

## DESCRIPTION OF THE INVENTION

### Brief description of the invention

The present invention refers to a method for the treatment, diagnosis and/or prognosis of mitochondrial diseases (i.e., pathologies associated with mitochondrial dysfunction), preferably for the treatment, diagnosis and/or prognosis of neurodegenerative mitochondrial diseases (i.e., treatment, diagnosis and/or prognosis of neuroinflammation and/or neurodegeneration in the context of those mitochondrial diseases).

The inventors of the present invention has discovered an unanticipated role of the mitochondrial ETC in maintaining NAPDH (Nicotinamide adenine dinucleotide phosphate) levels when PPP is compromised with prominent implications both *in vitro* and *in vivo*. This adaptive mechanism involves the rewiring of the TCA (tricarboxylic acid) cycle to maintain production of malate and subsequent generation of NADPH by the Malic enzyme 1 (ME1), which is a general phenomenon conserved across multiple cell lines. Finally, the inventors of the present invention provide compelling evidence suggesting that this process underlies the differential sensitivity to ETC inhibition between neurons and astrocytes, the decline in NAPDH levels rather than ATP is responsible for reduced cellular fitness in ETC-impaired cells, which might have implications in the management of mitochondrial diseases.

Particularly, the inventors of the present invention have uncovered that, upon ETC inhibition, a non-canonical TCA cycle is upregulated to maintain malate levels and concomitant production of NADPH. They found that Pyruvate carboxylase (PC) and ME1, the key mediators of this metabolic reprogramming, are selectively expressed in astrocytes compared to neurons, underlining their differential sensitivity to ETC inhibition. Moreover, the inventors of the present invention demonstrate that the observed phenotype in these brain cells upon ETC inhibition is specifically attributed to a decline in NADPH levels, rather than ATP.

So, the inventors of the present invention, by augmenting NADPH levels in the brain, for example by increasing the expression and/or activity of enzymes that produce NADPH like ME1, isocitrate dehydrogenase 1 (IDH1), or glucose 6 phosphate dehydrogenase (G6PD), suppress neuroinflammation and corrects motor function and coordination in a preclinical mouse model of CI deficiency, using a model of Leigh syndrome (a severe neurological disorder) with impaired CI activity as proof of concept. These studies provide a cohesive explanation for the differential sensitivities of distinct types of brain cells to ETC inhibition, which might have profound implications in the management of mitochondrial diseases.

So, the first embodiment of the present invention refers to NADPH for use in a method for the treatment of mitochondrial diseases, preferably the treatment of neurodegenerative mitochondrial diseases. See for instance [DiMauro S, Schon EA, Carelli V, Hirano M. The clinical maze of mitochondrial neurology. Nat Rev Neurol. 2013;9(8):429-444. doi:10.10381nrneurol.2013.126] dealing with mitochondrial neurology or [Gorman GS, Chinnery PF, DiMauro S, et al. Mitochondrial diseases. Nat Rev Dis Primers. 2016;2:16080. Published 2016 Oct 20. doi:10.1038/nrdp.2016.80] explaining that mitochondrial diseases are the most common group of inherited metabolic disorders and are among the most common forms of inherited neurological disorders.

In a preferred embodiment of the present invention, the method comprises promoting the production of NADPH by increasing the expression and/or activity of enzymes that produce NADPH.

In a preferred embodiment of the present invention, the method comprises promoting the production of NADPH by increasing the expression and/or activity of enzymes that produce NADPH, preferably by means of the administration of a vector to deliver and enhance the expression and/or activity of enzymes that produce NADPH in the brain or by means of the administration of molecule able to enhance the expression and/or activity of enzymes that produce NADPH in the brain.

In a preferred embodiment of the present invention, the vector is an adeno-associated virus with tropism to the central nervous system.

In a preferred embodiment of the present invention, the enzyme that produce NADPH is selected from ME1, IDH1 or G6PD.

The second embodiment of the present invention refers to an *in vitro* method for the diagnosis and/or prognosis of mitochondrial diseases, preferably neurodegenerative mitochondrial diseases, which comprises assessing the level of NADPH, or of the expression level and/or activity of enzymes that produce NADPH, in a biological sample obtained from the subject. Alternatively, the present invention refers to the *in vitro* use of NADPH, or of an enzyme that produce NADPH, or of a kit comprising reagents for the identification of NADPH, or of an enzyme that produce NADPH, for the diagnosis and/or prognosis of mitochondrial diseases, preferably neurodegenerative mitochondrial diseases.

In a preferred embodiment of the present invention, the diagnosis and/or prognosis method comprises assessing the level of expression and/or activity of enzymes that produce NADPH, in a biological sample obtained from the subject, selected from ME1, IDH1 or G6PD.

In a preferred embodiment of the present invention, the diagnosis and/or prognosis method comprises assessing the level of NADPH or the expression level and/or activity of enzymes that produce NADPH, in a biological sample obtained from the subject, wherein a lower level of NADPH or a lower level of expression and/or activity of enzymes that produce NADPH, as compared to a pre-established threshold level determined in control subjects who are not suffering from mitochondrial diseases, is an indication that the subject has a mitochondrial disease or has a poor prognosis.

In a preferred embodiment of the present invention, the biological sample is selected from tissue, blood, serum, plasma or cerebrospinal fluid.

For the purpose of the resent invention, the following terms are defined:

- The term "comprising" means including, but not limited to, whatever follows the word "comprising". Thus, use of the term "comprising" indicates that the listed elements are required or mandatory, but that other elements are optional and may or may not be present.

- The term "consisting of" means including, and limited to, whatever follows the phrase "consisting of". Thus, the phrase "consisting of" indicates that the listed elements are required or mandatory, and that no other elements may be present.

- According to the present invention, a reference value can be a "pre-established threshold value" or a "cut-off' value. Typically, a "threshold value" or "cut-off value" can be determined experimentally, empirically, or theoretically. According to the present invention, the "pre-established threshold" value refers to a value previously determined in control subjects who are not suffering from mitochondrial diseases. Thus, for instance, the subject is likely to suffer from mitochondrial diseases, or to have a poor prognosis, when a lower level of NADPH or a lower level of expression and/or activity of enzymes that produce NADPH, as compared to a pre-established threshold level determined in control subjects who are not suffering from mitochondrial diseases, is identified. A "threshold value" can also be arbitrarily selected based upon the existing experimental and/or clinical conditions, as would be recognized by a person of ordinary skilled in the art. The "threshold value" has to be determined in order to obtain the optimal sensitivity and specificity according to the function of the test and the benefit/risk balance (clinical consequences of false positive and false negative). Typically, the optimal sensitivity and specificity (and so the "threshold value") can be determined using a Receiver Operating Characteristic (ROC) curve based on experimental data.

**Brief description of the figures**

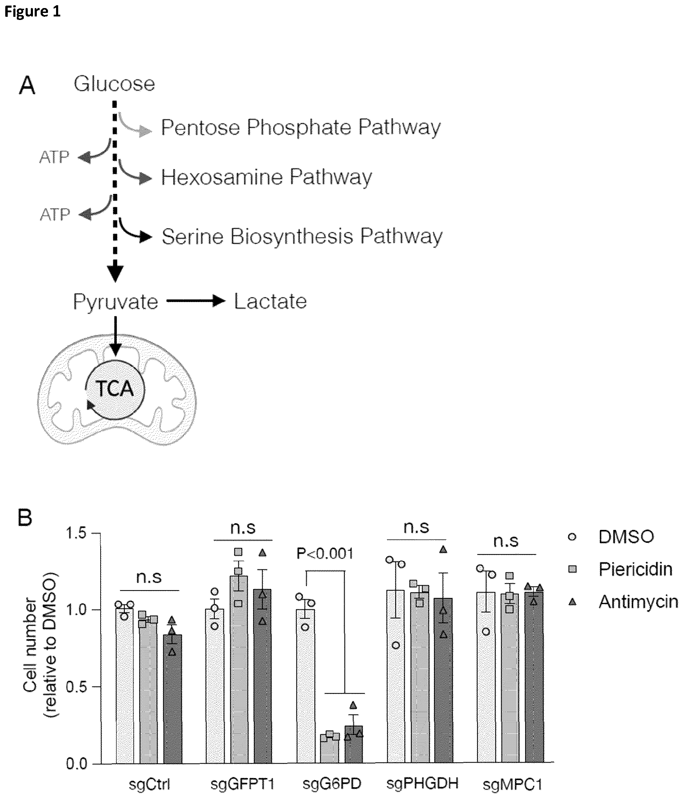

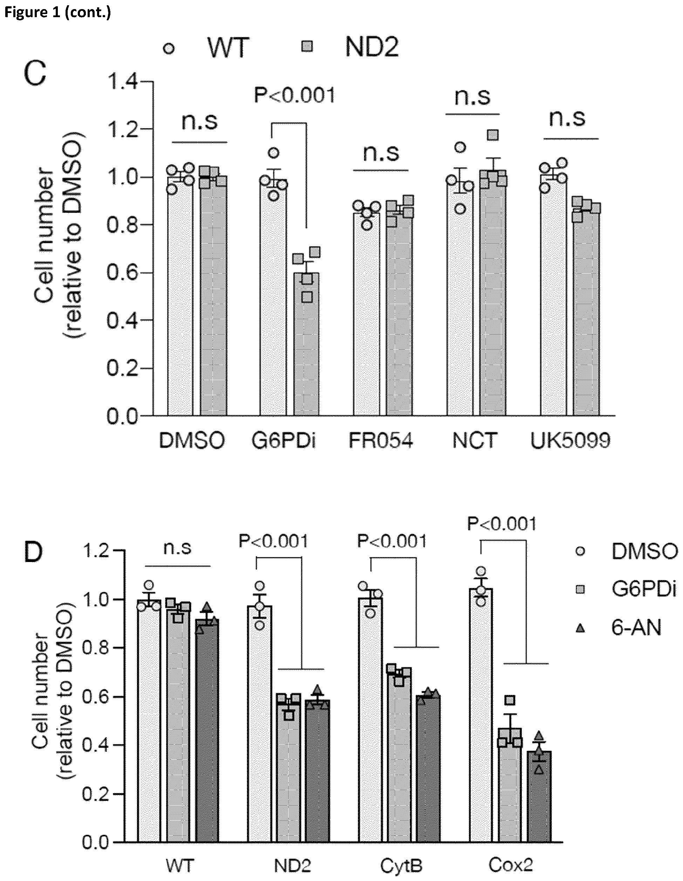

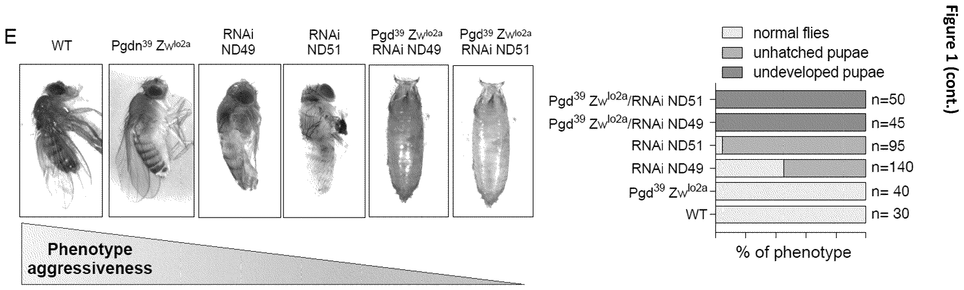

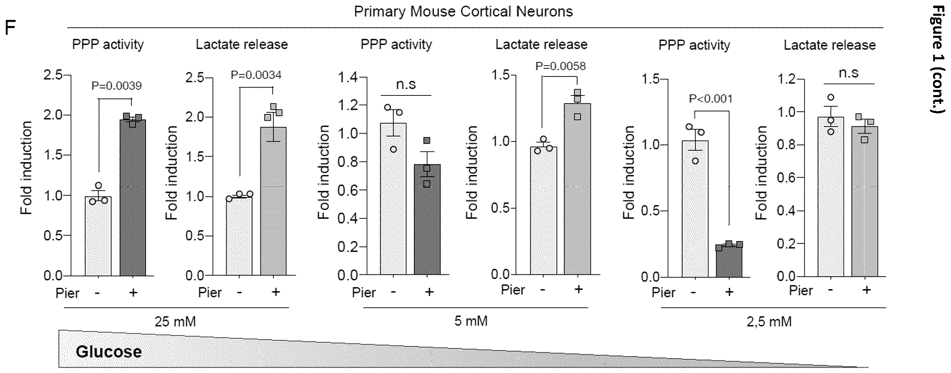

**Figure 1. Cells and tissues with mitochondrial dysfunction rely on PPP activity for survival. (A)** Scheme illustrating metabolic pathways that rely on glucose. **(B)** Cell number of the indicated CRISPR-mediated knock-out cells treated either with complex I inhibitor Piericidin (100 nM) or Antimycin (500 nM) for 72 h (n=3). **(C)** Cell number at 72 h of WT and ND2 mutant cybrid cells treated with inhibitors that selectively target the following pathways: G6PDi inhibits PPP (50 uM), FR054 (100 uM) inhibits the hexosamine biosynthetic pathway, NCT-503 (10 uM) inhibits the serine biosynthesis pathway and UK5099 (10uM) inhibits the mitochondrial pyruvate carrier (n=4). **(D)** Cell number at 72 h of WT, ND2, CytB and Cox2 mutant cybrid cells treated with the PPP inhibitors G6PDi (50 uM) and 6-Aminonicotinamide (6AN) (5 uM) (n=4). **(E)** Representative photographs of WT and mutant flies showing the increased aggressiveness of the phenotype (left) and subsequent quantification (right). **(F)** Analysis of PPP activity and lactate secretion as a proxy for glycolytic flux in primary mouse cortical neurons cultured under 25 mM, 5mM or 2,5 mM D-glucose media that have been treated with Piericidin (100 nM) for 16 h (n=3). Data are presented as means $\pm$ SEM. The statistical significance of the differences between groups was determined by two-way ANOVA (**B, C** and **D**) and paired two-tailed Student's t test **(F)**. n.s not significant. Abbreviations: Pier, Piericidin; 6-AN, 6-Aminonicotinamide.

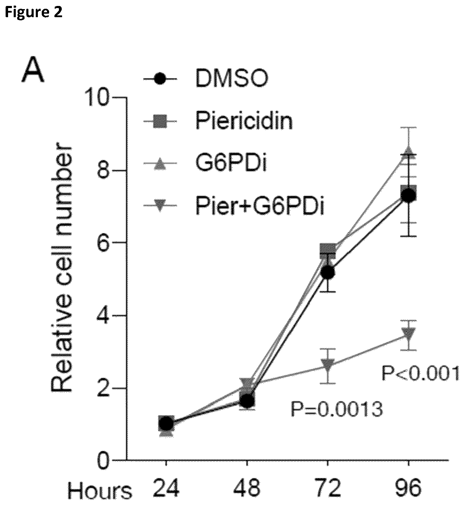

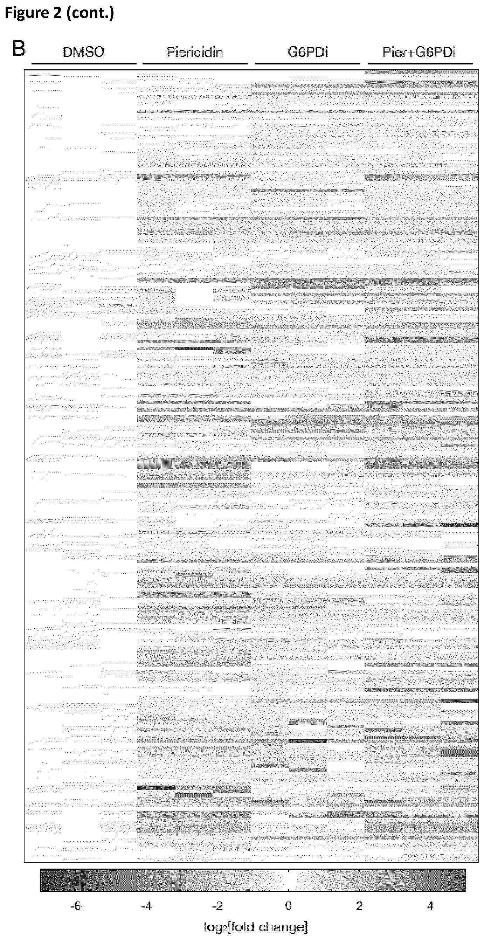

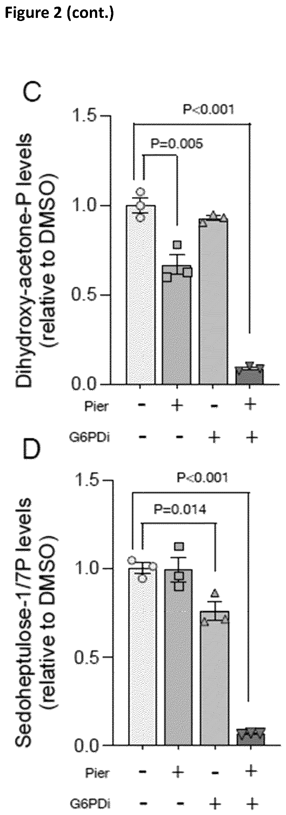

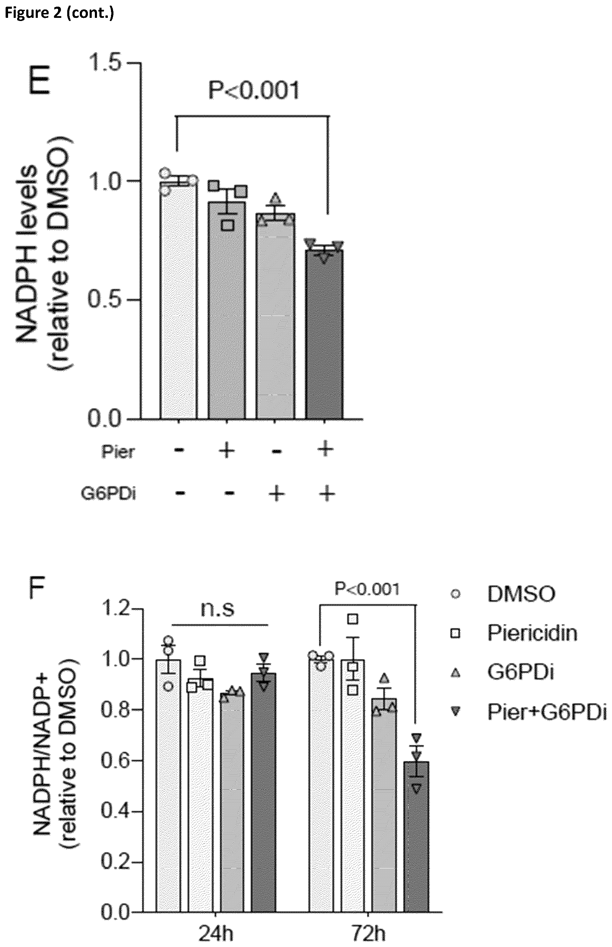

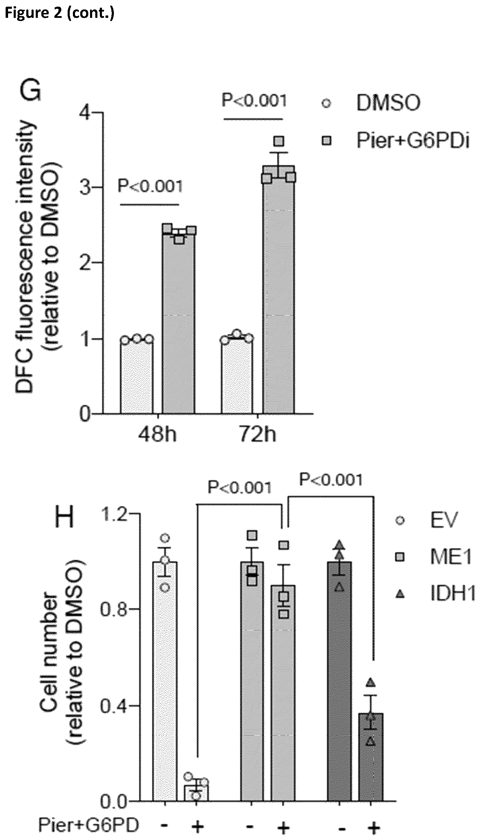

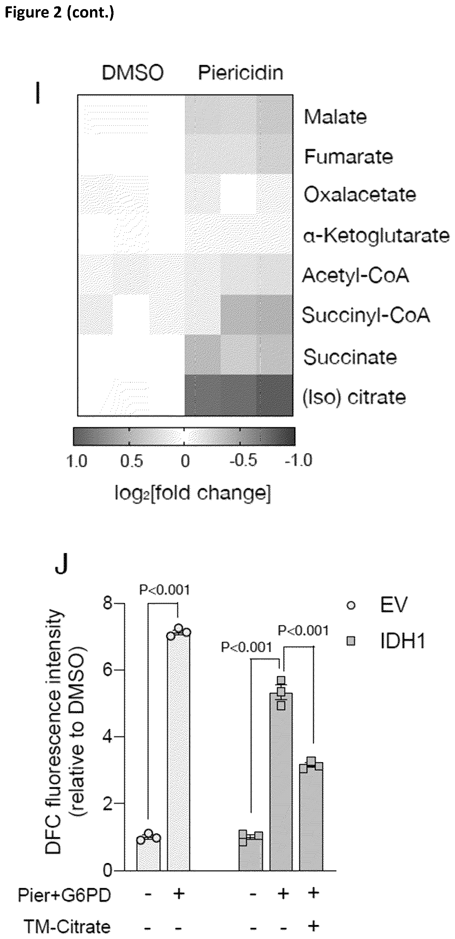

**Figure 2. Synthetic lethality between PPP and OXPHOS is reduced by increased ME1 expression. (A)** Growth curves of 143B cells treated with DMSO, Piericidin (100 nM), G6PDi PPP (50 uM), and the combination of both (n=3). **(B-E)** Heat map showing intracellular levels of water-soluble metabolites in 143B cells treated with the indicated inhibitors for 48 h **(B)** and relative levels of dihydroxyacetone phosphate **(C)**, D-sedoheptulose-1,7-bisphosphate **(D)** and NADPH **(E)** (n=3). **(F)** NADPH/NADP+ ratio at 24 and 72 h in 143B cells treated with DMSO, Piericidin (100 nM), G6PDi PPP (50 uM), and the combination of both (n=3). **(G)** Relative reactive oxygen species (ROS) levels measured using dichlorodihydrofluorescein diacetate (H2DCFDA) in 143B cells treated with DMSO or Piericidin (100 nM) plus G6PDi (50 uM) (n=3). **(H)** Cell number at 72 h in 143B cells overexpressing ME1 or IDH1 and treated with DMSO or Piericidin (100 nM) plus G6PDi (50 uM) (n=3). **(I)** Heat map showing intracellular levels of TCA cycle associated metabolites analyzed by LC-MS in DMSO-treated or Piericidin-treated (100 nM) 143B cells for 24 h (n=3). **(J)** Relative reactive oxygen species (ROS) levels in WT and IDH1 overexpressing 143B cells treated with DMSO or Piericidin (100 nM) plus G6PDi (50 uM) and supplemented with 2 mM trimethyl citrate (n=3). Data are presented as means ± SEM. The statistical significance of the differences between groups was determined by two-wayANOVA (**A, C, D, E, F, G, H** and **J**). n.s not significant. Abbreviations: Pier, Piericidin; TM-Citrate, Trimethyl citrate.

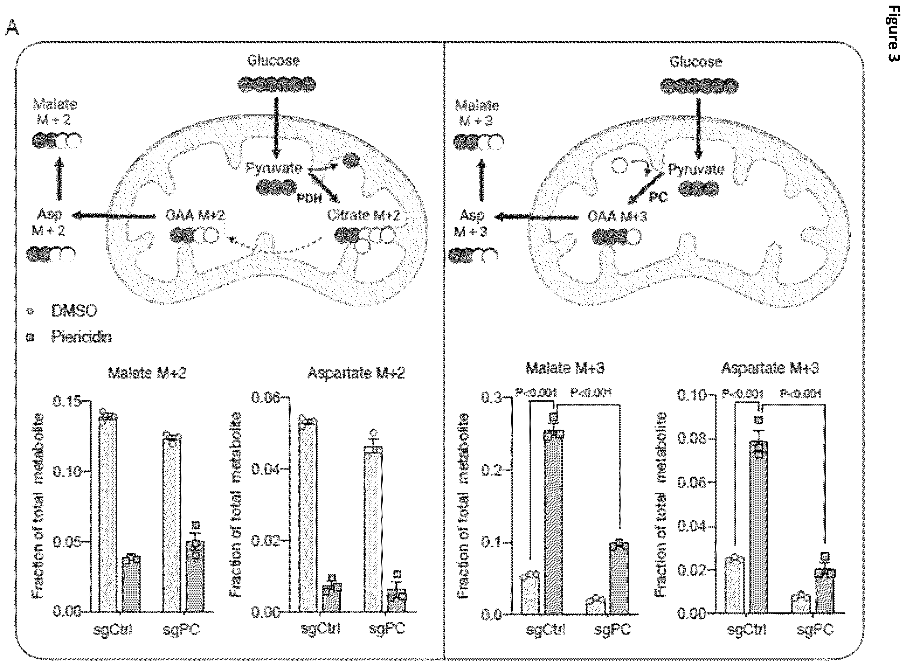

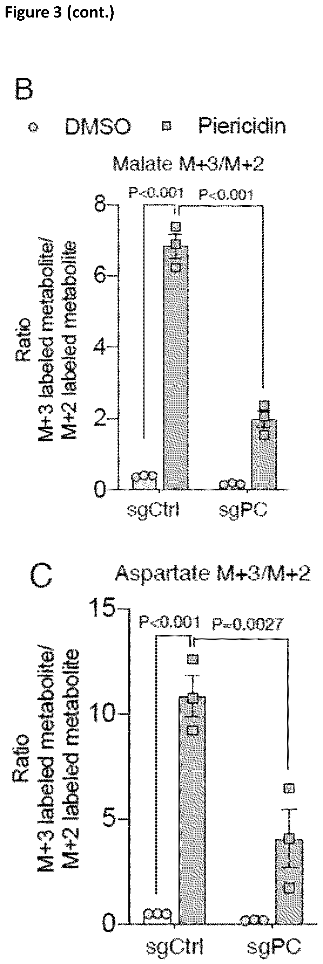

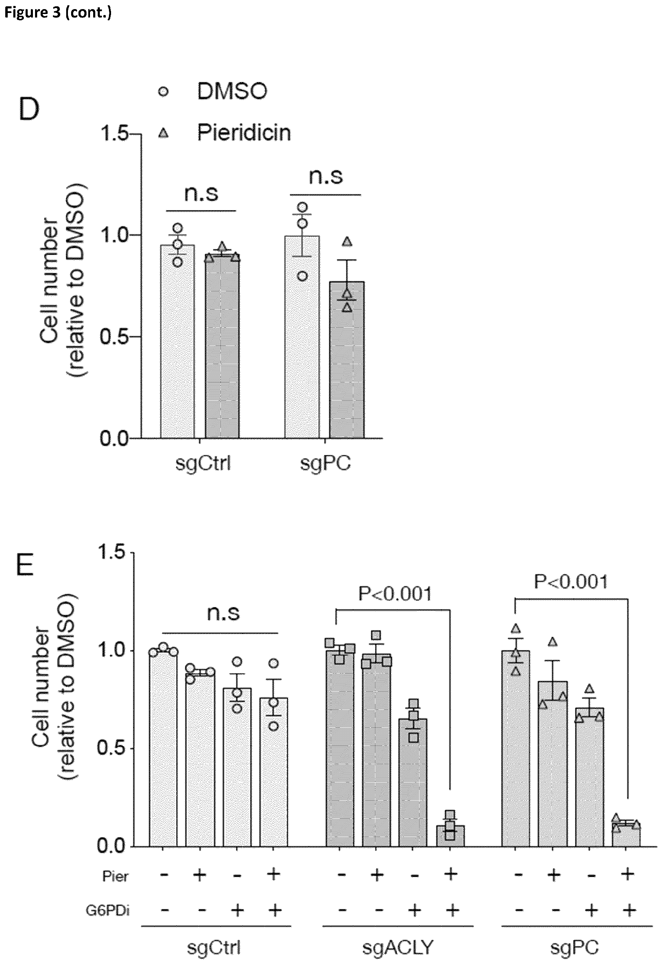

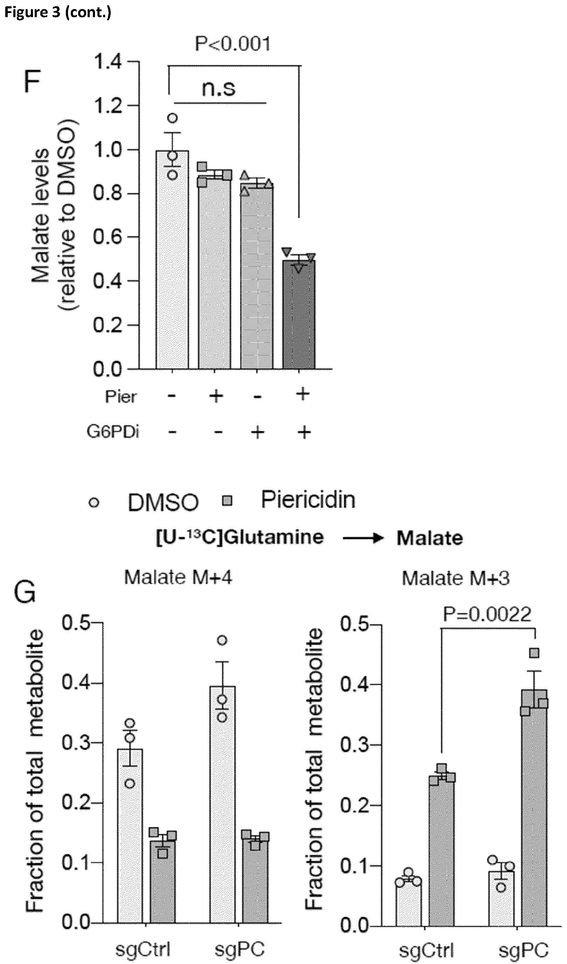

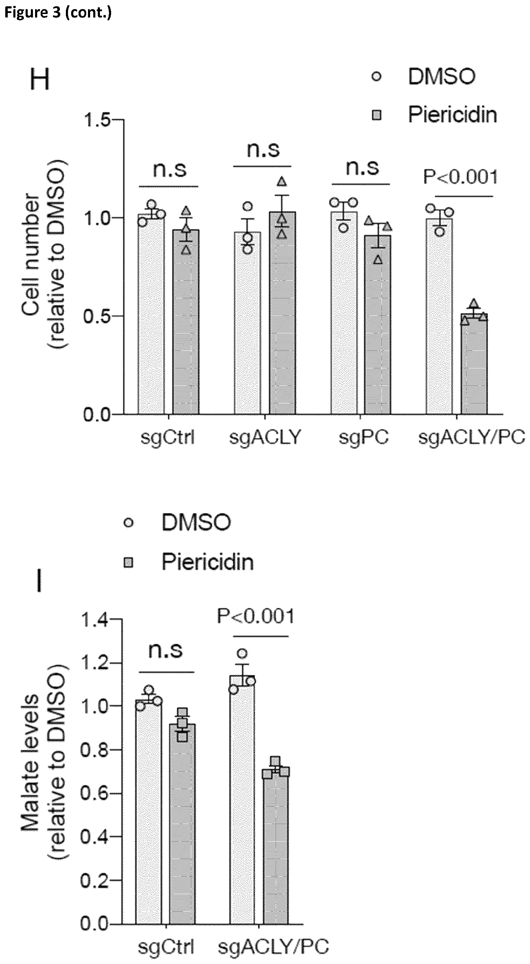

**Figure 3. CI inhibition elicits a metabolic reprogramming via PC and ACLY to enable the generation of malate and NADPH. (A)** Model illustrating the fate of uniformly labeled 13Cglucose after entering a fully functional TCA cycle (DMSO condition) or a truncated TCA cycle (Piericidin condition). CI inhibition decreases malate and aspartate M+2 forms originated by oxidation of glucose-derived pyruvate in the TCA cycle and increases malate and aspartate M+3 forms coming from carboxylation of pyruvate by the PC enzyme (n=3). **(B-C)** M+3/M+2 ratios of malate **(B)** and aspartate **(C),** which represent the proportional contribution of PC vs PDH to metabolite labeling (n=3). **(D)** Cell number of sgCtrl and sgPC 143B cells treated with DMSO or Piericidin (100 nM) for 72 h (n=3). **(E)** Cell number of sgCtrl, sgACLY and sgPC treated with the indicated inhibitors for 72 h (n=3). **(F)** Levels of intracellular malate in 143B cells treated with the indicated inhibitors for 48 h (n=3). **(G)** Labelling of M+4 and M+3 from uniformly labeled 13Cglutamine in sgCtrl and sgPC 143B cells treated with DMSO or Piericidin (100 nM) for 24 h (n=3). **(H)** Cell number of sgCtrl, sgACLY, sgPC and double KO sgACLY/PC 143B cells treated with DMSO or Piericidin (100 nM) for 72 h (n=3). **(I)** Levels of intracellular malate in sgCtrl and double KO sgACLY/PC 143B cells treated with DMSO or Piericidin (100 nM) for 72 h (n=3). Data are presented as means ± SEM. The statistical significance of the differences between groups was determined by two-wayANOVA (A, **B, C, D, E, F, G, H** and **I**). n.s not significant.

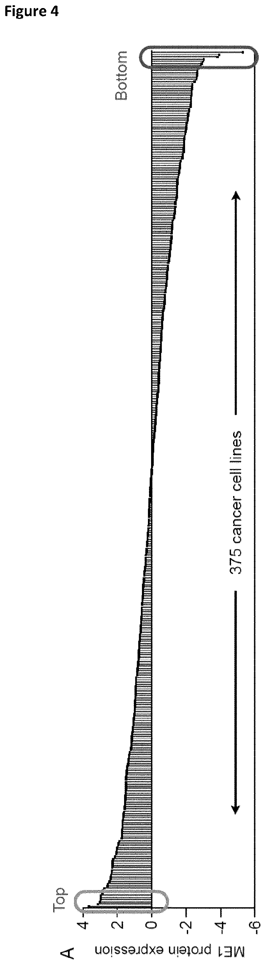

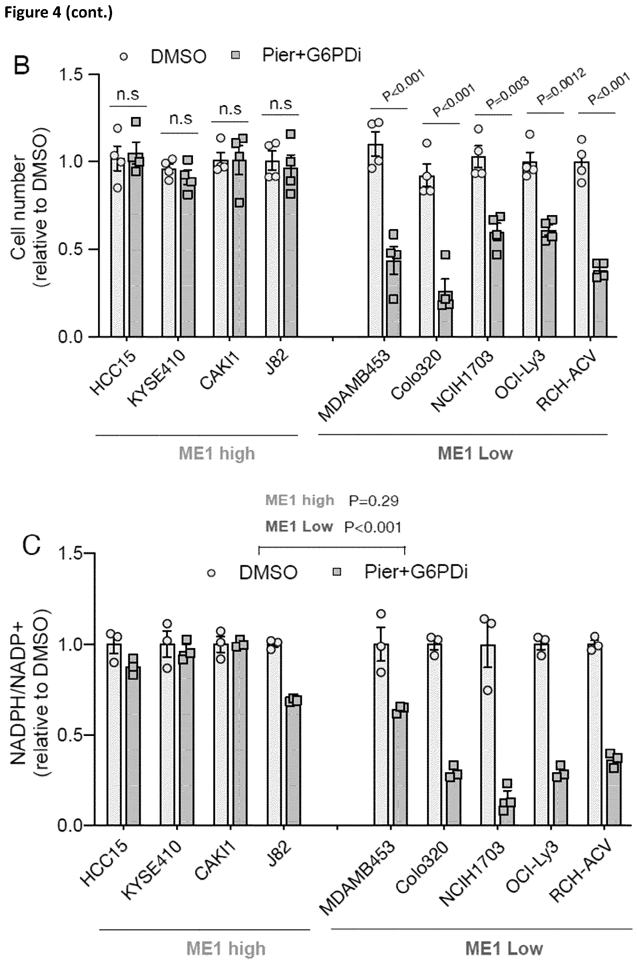

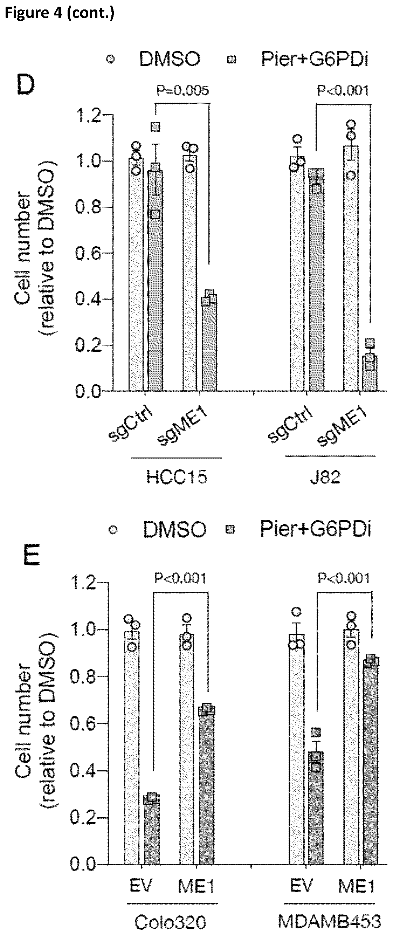

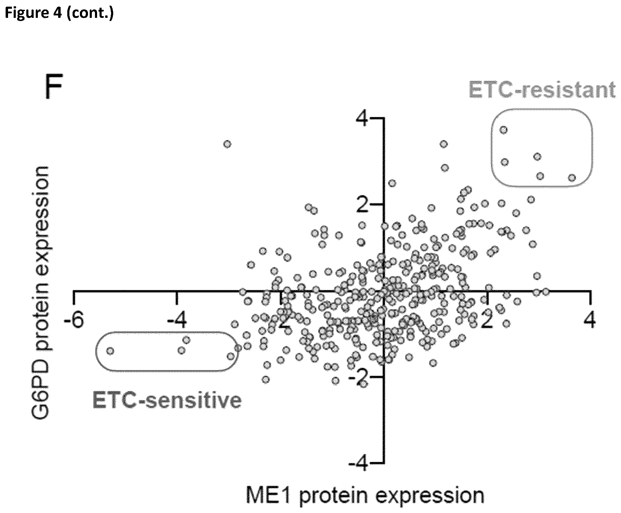

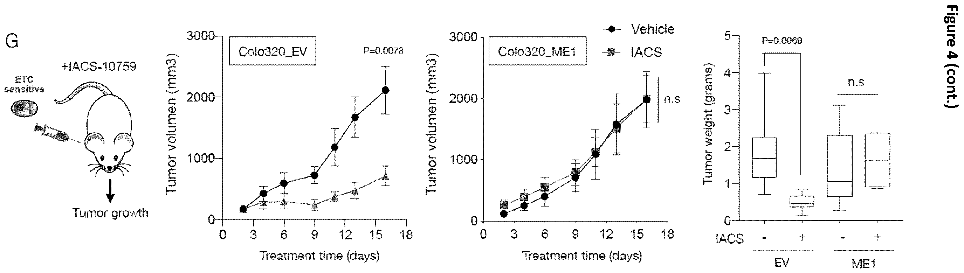

**Figure 4. Cancer sensitivity to OXPHOS inhibitors is determined by ME1 expression levels. (A)** In silico analysis of ME1 protein expression extracted from mass spectrometry data across 375 cell line collections including the Cancer Cell Line Encyclopedia (CCLE)26. **(B-C)** Cell number **(B)** and NADPH/NADP+ ratio (C) in ME1 high and ME1 low cell lines treated with DMSO or Piericidin (100 nM) plus G6PDi (50 uM) for 96 h (n=4 and n=3). (D-E) Cell number in Piericidin plus G6PDi treated ME1 high cell lines HCC15 and J82 where ME1 has been depleted by CRISPR **(D)** and ME1 low cell lines where ME1 has been ectopically overexpress **(E)** (n=3). **(F)** Analysis correlating the protein expression of NADPH generating enzymes G6PD and ME1 in hundreds of cell lines. Highlighted in red are cell predicted to be sensitive to ETC inhibition whereas in blue are those predicted to be insensitive. **(G)** Xenograft experiments using ME1 low Colo320 cells. Evolution of tumor growth (left) and final tumor weight (right) in mice injected with WT or ME1 overexpressing Colo320 and treated with vehicle or CI inhibitor IACS-10759 every other day (n=7). Data are presented as means ± SEM. The statistical significance of the differences between groups was determined by two-way ANOVA (**B, C, D E** and **G**). n.s not significant. Abbreviations: Pier, Piericidin; ETC, Electron Transport Chain; EV, empty vector.

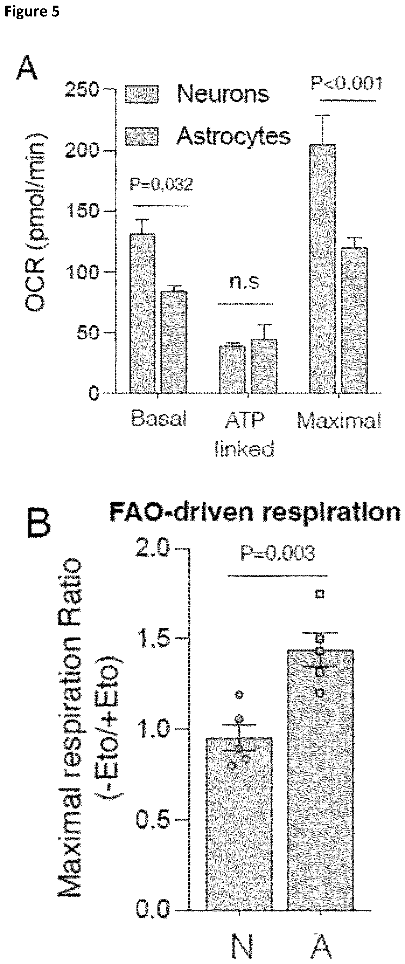

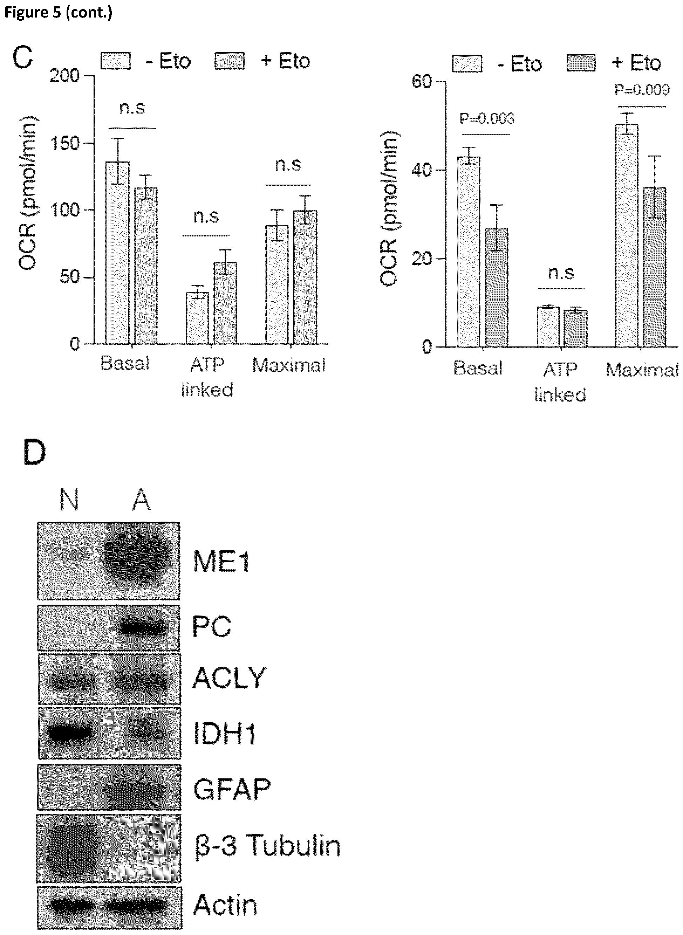

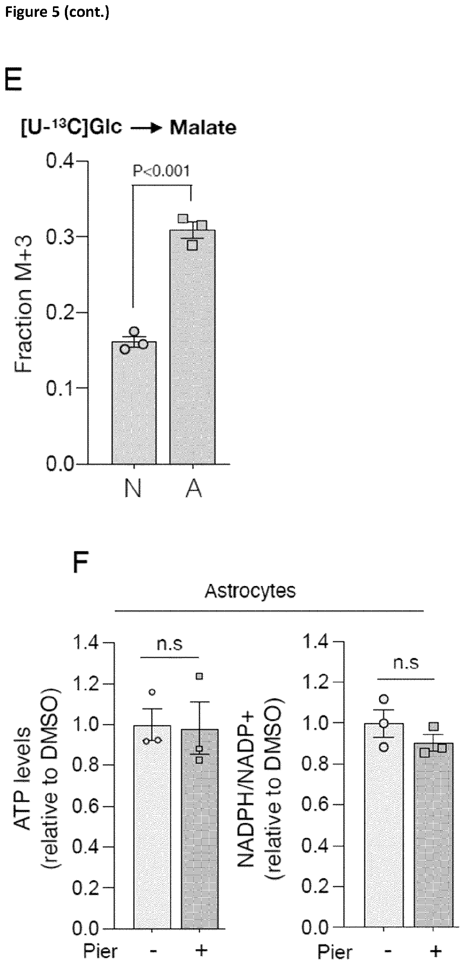

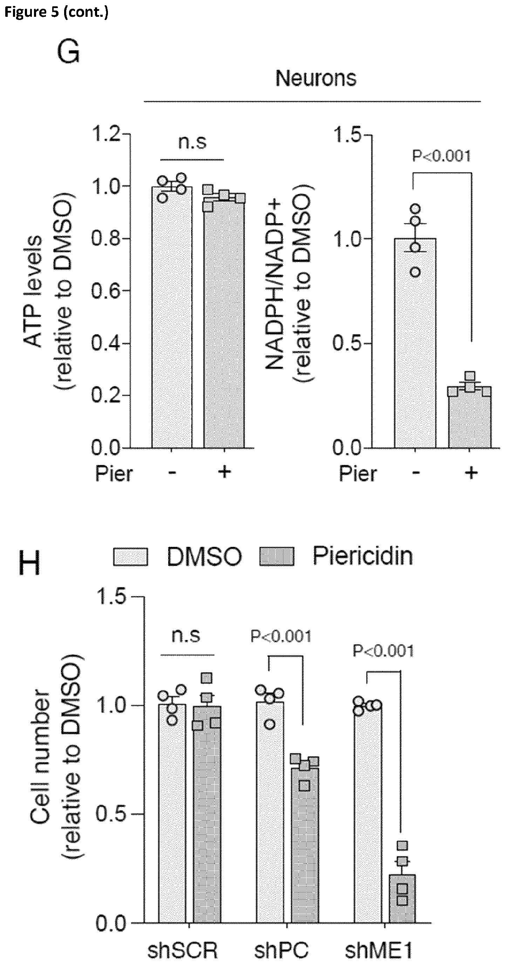

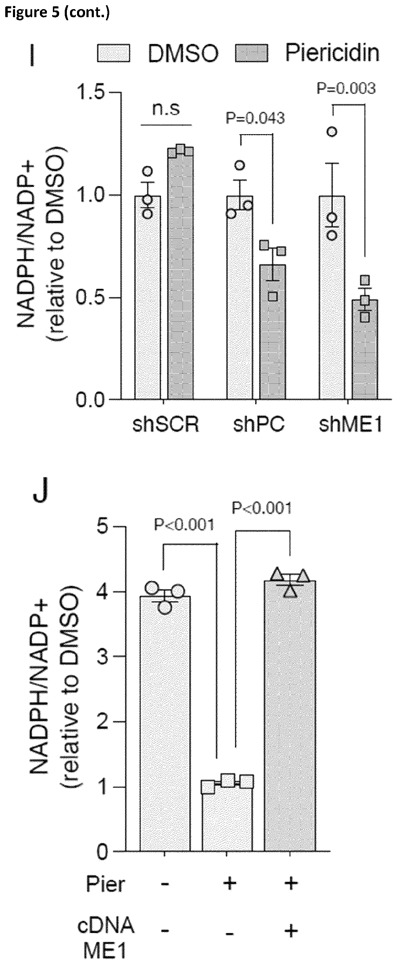

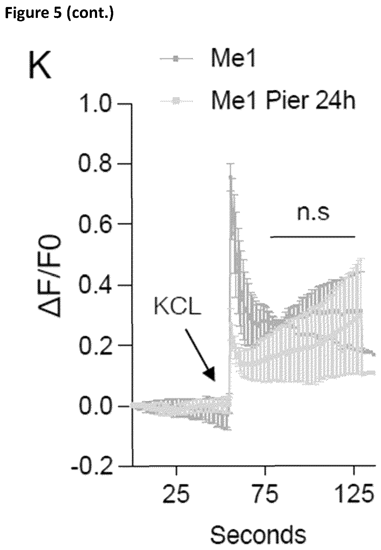

**Figure 5. Distinct Responses to Mitochondrial Complex I Inhibition in Astrocytes and Neurons. (A-B)** Seahorse Mito Stress Test analysis in neurons and astrocytes using Seahorse XF DMEM assay medium containing glucose, pyruvate and glutamine **(A)** or palmitate-BSA plus L-carnitine as substrates for respiration **(B).** FAO-driven respiration in neurons and astrocytes is calculated as the ratio between maximal respiration (FCCP) without etomoxir and with the addition of etomoxir, prior to the assay. **(C)** Seahorse XF Mito Stress Test assay in neurons (left) and astrocytes (right) that were incubated with etomoxir for 30 mins prior the analysis, revealing distinct dependencies in FAO-driven respiration. **(D)** Inmunoblot showing expression of the indicated proteins in neurons (N) and astrocytes (A). GFAP was used as a specific marker for astrocytes whereas Beta Tubulin III was used as a marker for neurons. **(E)** Labelling of M+3 malate coming from uniformly labeled 13Cglutamine in neurons (N) and astrocytes (A). **(F-G)** ATP levels and NADPH/NADP+ ratio in astrocytes **(F)** and neurons **(G)** treated with DMSO or Piericidin (100 nM) for 24 h. **(H-I)** Cell number **(H)** and NADPH/NADP+ ratio (I) in astrocytes, after ME1 or PC knockdown, that were treated either with DMSO or Piericidin (100nM) for 96 h. **(J)** NADPH/NADP+ ratio in neurons overexpressing ME1 and treated with DMSO or Piericidin (100 nM) for 24 h. **(K)** Calcium response after stimulation with potassium chloride (KCl) in neurons overexpressing ME1 and treated with DMSO or Piericidin (100 nM) for 24 h. Data are presented as means ± SEM. The statistical significance of the differences between groups was determined by paired two-tailed Student's t test (B, E, F and G) and two-way ANOVA (A, C, H, I and J). n.s not significant. Immunoblots shown are representative of >3 independent experiments. Abbreviations: N, Neurons; A, Astrocytes; Pier, Piericidin; KCL, potassium chloride.

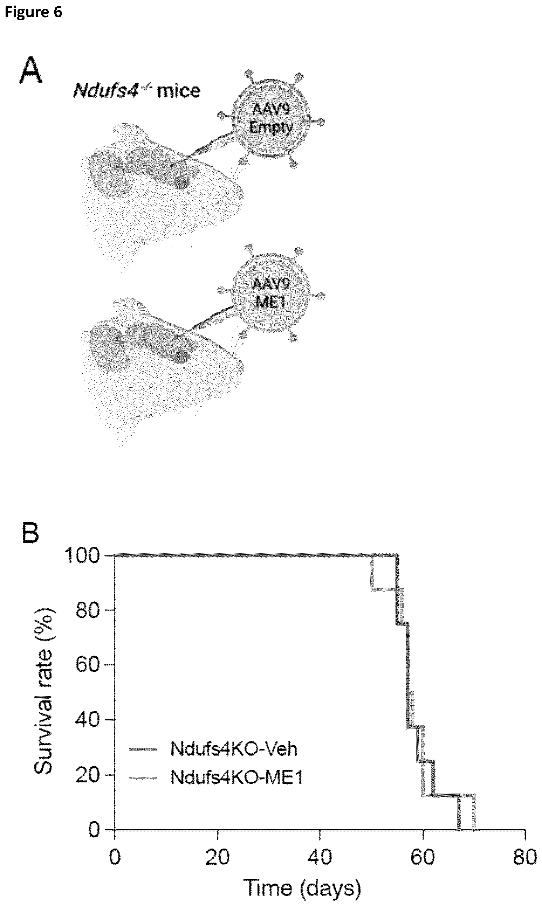

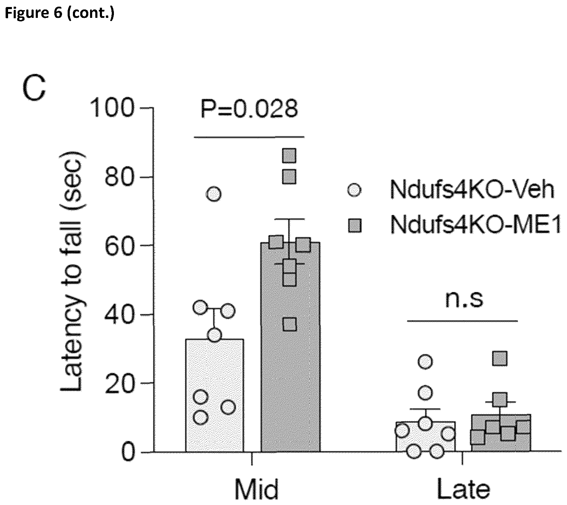

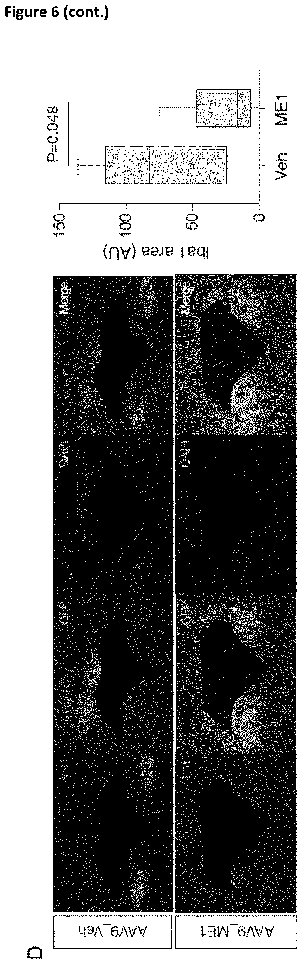

**Figure 6. Boosting ME1 Function Alleviates Neuroinflammation and Enhances Motor Coordination in Ndufs4-/- Mice. (A)** Schematic of delivering ME1 to the vestibular nuclei of Ndufs4-/- mice via stereotaxic injections. **(B)** Survival of Ndufs4-/- mice injected with AAV9-Veh or AAV9-ME1. (n=8). **(C)** Rotarod performance of AAV9-Veh or AAV9-ME1 Ndufs4-/- injected mice, at 40 (Mid) and 50 days (Late). (n=7). **(D)** Representative images and quantification of Iba-1 staining in the vestibular nucleus (VN) region of the brain. (n=6). Data are presented as means $\pm$ SEM. The statistical significance of the differences between groups was determined by one-way ANOVA with the Tukey-Kramer test (A, B and C) and Welch's t-test **(D)**. n.s not significant. Images shown are representative of >3 independent experiments. Abbreviations: Veh, Vehicle.

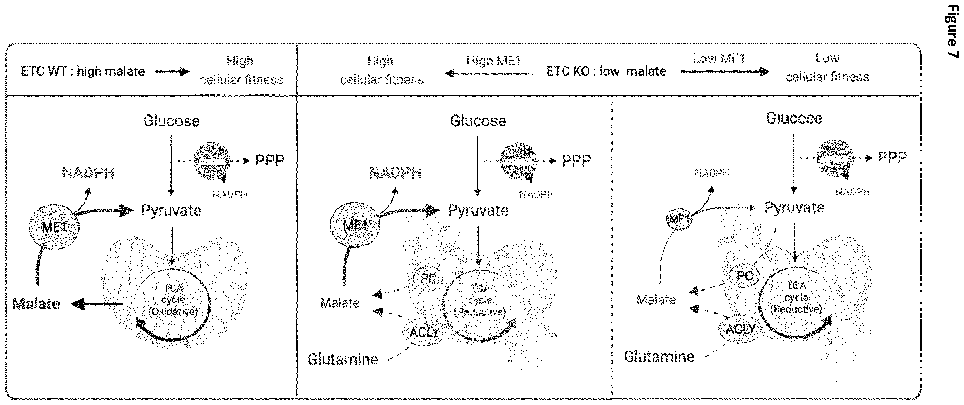

**Figure 7. Model describing the role of mitochondria in maintaining NADPH homeostasis.** Proposed model showing how mitochondrial-derived malate in intact cells, can sustain NADPH levels in the absence of optimal PPP activity. Conversely, cells with severe impairments in the ETC redirect pyruvate and/or glutamine through non-canonical TCA cycle as sources to generate malate. Under these conditions, ETC-deficient cells heavily rely on ME1 expression to maintain NADPH levels and ensure cellular fitness.

## Detailed description of the invention

The present invention is illustrated by means of the Examples set below without the intention of limiting its scope of protection.

## Example 1. Material and Methods

### Example 1.1. Cell Culture and Treatments

All cell lines, 143b, HeLa, U2OS, and transmitochondrial cybrids carrying mutation in CI (ND2), CIII (CytB) or CIV (Cox2) subunits, were maintained in DMEM high glucose (HyClone), 10% FBS and 1% P/S at 37°C with 5% $CO_2$. Mitochondrial mutant cells were cultured with supplementation of 50 $\mu$g/mL uridine. The selected cancer cell lines with different levels of malic enzyme (HCC15, KYSE410, CAKI1, J82, MDAMB453, Colo320, NCIH1703) were obtained from the DSMZ-German Collection of Microorganisms and Cell Cultures GmbH and cultured in the same DMEM 10% FBS medium. OCI-Lys3 and RCH-ACV cell lines (cells in suspension), were obtained from the same repository and cultured in RPMI 10% FBS medium. Transfections for gain- and loss-of-function studies were performed according to the manufacturer's instructions using the polyfect reagent (Qiagen, 301107). For cell number and proliferation experiments, cells were seeded in 12 or 6-well plates and allowed to proliferate for the specified durations. Subsequently, cells were trypsinized and quantified using a hemocytometer (NanoEnTek, DHC-N01). Cell viability was quantified using propidium iodide (PI; 50 $\mu$g/ml) staining.

### Example 1.2. Isolation of mouse primary neurons and astrocytes

Primary cultures of C57BL/6 mouse cortical neurons were prepared from embryos between embryonic days 16.5 and 17.5 and seeded onto pre-treated plates coated with Poly-L-lysine hydrobromide (500 $\mu$g/ml). We used Neurobasal medium without D-glucose and sodium pyruvate (ThermoFisher) to modulate D-glucose levels, depending on the experiment (2.5 mM, 5 mM, or 25 mM).
In standard experiments, we utilized Neurobasal medium with 25 mM D-glucose, 1 mM sodium pyruvate (ThermoFisher), B27 plus supplement (GIBCO), GlutaMax I 100X (GIBCO), and 1% penicillin/streptomycin (P/S). Starting from day 6 *in vitro* (DIV6), half of the medium was replaced every 3-4 days, omitting GlutaMax I.
Primary cultures of C57BL/6 mouse cortical astrocytes were derived from postnatal day 2-3 pups. They were plated on pre-treated dishes coated with Poly-L-lysine (100 $\mu$g/ml) and cultured in DMEM/F-12 (ThermoFisher) supplemented with 15% FBS and 1% penicillin/streptomycin (P/S). After 24 hours of plating, the medium was switched to 10% FBS.

### Example 1.3. Generation of knockout and knockdown cell lines

Generation of knockout cell lines using CRISPR-Cas9 technology was performed with the lenti-CRISPR v2 vector (Addgene #52961). Guide sequences are:

sgGFPT1, 5-TTGGAATAGCTCATACCCGT-3 (SEQ ID NO: 1);

sgG6PD, 5-CTTGAAGGTGAGGATAACGC-3 (SEQ ID NO: 2);

sgPHGDH, 5-GACACACCTACCTGTCGTGG-3 (SEQ ID NO: 3);

sgMPC1, 5-TGTCAAAGAATAGCAACAGA-3 (SEQ ID NO: 4)

sgPC, 5-CAGGCCCGGAACACACGGA-3 (SEQ ID NO: 5);

sgACLY, 5-GCCAGCGGGAGCACATCGGT-3 (SEQ ID NO: 6);

sgME1, 5-ATAGAGCCAATTTACCCACA-3 (SEQ ID NO: 7).

## Example 1.4. shRNAs and Overexpressing Vectors in primary cultures

To downregulate ME1 and PCx in primary astrocyte cultures, we employed Sigma-designed shRNA sequences: TRCN0000375960 (for ME1, achieving a 91% knockdown) and TRCN0000112425 (for PCx, resulting in a 96% knockout). These shRNAs were delivered using the TRC2 vector with puromycin resistance. Astrocytes were plated at a density of 20,000 cells/ml and transduced when they reached a monolayer state, with the addition of 200µl of virus per ml. Infected astrocytes were then subjected to a 7-day selection period with puromycin (1,5 µg/ml) before commencing the experiments.

## Example 1.5. NADPH/NADP+ levels

The NADP/NADP-Glo™ Assay (Promega) was employed to assess the NADP/NADPH ratio in cultured cells, following the manufacturer's instructions (Technical Bulletin Number TM400). This bioluminescent assay can detect both total oxidized and reduced nicotinamide adenine dinucleotide phosphates (NADP+ and NADPH) and determine their ratio.

Cells were cultured in 24-well plates (Falcon®) at varying concentrations depending on the cell type and the specific experiment. After removing the media, we added 100 µl of PBS 1X per well and lysed the cells by introducing 100 µl of a base solution with 1% DTAB (1:1 ratio). To adapt the volumes for a 96-well plate, we divided the 200 µl total into two parts, allocating 50 µl for measuring NADP+ and 50 µl for NADPH in separate positions on the plate, allowing us to measure both NADP and NADPH levels. The assay was then carried out as per the standard protocol. Luminescence was measured using the CLARIOstar Plus plate reader from BMG LABTECH.

## Example 1.6. ROS measurements

ROS levels were assessed *in vitro* using the fluorescent probe CM-H2DCFDA (Invitrogen) at a concentration of 5 µM dissolved in DMSO. Cells were incubated in their original medium with the fluorescent probe for 20 minutes, after which the medium was replaced with PBS, and the fluorescence was quantified using the CLARIOstar Plus plate reader (BMG LABTECH) (492-495/517-527 nm). To standardize the data, we normalized it relative to the number of cells, determined using Hoechst staining. As positive controls, we exposed cells to 0.6% $H_2O_2$ (approximately 20 µM) for 5 minutes following incubation with CM-H2DCFDA. For negative controls, we utilized wild-type cells without the dye, both with and without Hoechst staining.

## Example 1.7. Fluorescence-based assay for detecting intracellular calcium mobilization

Neuronal functionality was measured by the Rhod-4 No Wash Calcium Assay Kit (Abcam, Cat No. ab112157), following the manufacturer's instructions for intracellular calcium assay. In short, culture medium was removed, and Rhod-4 dye containing buffer was gently added to cells, followed by incubation at 37 degrees for 30 min followed by 20 min at room temperature. For imaging and quantification of intracellular calcium mobilization, confocal images were acquired using a Zeiss LSM800 confocal microscope (Jena, Alemania) enabled with a 10X objective. Time lapse images of the confocal scan were acquired every 0.5 second over a ten-minute period. Neuronal cultures were treated with 100mM KCl (final concentration) added after the first 4 minutes to establish a baseline and observe the steady- state changes in calcium mobilization in response to the treatment. Acquired confocal images were post-processed using the free and open-source Fiji image processing software. Fluorescent intensity changes were quantified using fixed parameters across the entire set of captured images.

## Example 1.8. Patch-clamp capacitance analysis

A glass coverslip containing mixed cortical cultures of 10-14 DIV was placed in an immersion chamber constantly perfused with aCSF (119 mM NaCl, 2.5 mM KCl, 2.5 mM $CaCl_2$, 1.2 mM $MgCl_2$, 26 mM $NaHCO_3$, 1 mM $NaH_2PO_4$, 11 mM glucose pH adjusted to 7.4 and osmolarity to 290 ±5 mOsm) gassed with carbogen (5-10% $CO_2$, 90-95% $O_2$) and its temperature closely monitored at 29°C. Cells were recorded under current-clamp configuration with patch recording glass electrodes (4-6 MΩ) composed of silver/silver chloride electrodes inserted in glass pipettes filled with internal solution (115 mM K gluconate, 20 mM KCl, 10 mM HEPES, 2 mM $MgCl_2$, 4 mM $Na_2$-ATP, 0.3 mM $Na_3$-GTP pH adjusted to 7.2-7.3 and osmolarity to 290 ±5 mOsm). Resting membrane potential was directly acquired from these recordings. Voltage responses to current injections of -100 pA were used to calculate whole-cell input resistance and membrane capacitance. This last parameter is calculated from the exponential time constant (τ) of the voltage change, as τ=R*C (R: input resistance, C: capacitance).

Data acquisition was carried out with Multi-Clamp 700 A/B amplifiers and pCLAMP 9/10 software (Molecular Devices). Data analysis was performed using Clampfit software (Molecular Devices).

## Example 1.9. Immunoblot

Cells were harvested in RIPA buffer (10 mM Tris-HCl pH 8.0, 1 mM EDTA, 1% Triton X-100, 0.1% Sodium Deoxycholate, 0.1% SDS, 140 mM NaCl, 1X protease inhibitor cocktail, 1mM PMSF) and protein concentrations were quantified using the BCA assay (Pierce 23228). The following antibodies were used for western blot analysis: anti-ME1 (1/1000) (ab97445; Abcam); anti-G6PD (1/1000) (PA5-27359; Invitrogen); anti-PHGDH (1/1000) (14719-1-AP; Proteintech); anti-GFPT1 (1/1000) (14132-1-AP; Proteintech); anti-ACLY (1/1000) (15421-1-AP; Proteintech); anti MPC1 (1/1000) (14462; Proteintech); anti-PC (1/1000) (NBP-49536; Novus); anti-IDH1 (1/1000) (#8137; Cell Signaling); anti-B III tubulin (1/1000) (ab78078; Abcam); anti-B actin (1/2000) (A5441; Sigma); anti-GFAP (1/1000) (ab7260; Abcam).

### Example 1.10. Immunofluorescence

Neuron axon morphology was quantified in primary neurons exposed to DMSO or 100 nM piericidin for 24 h. Neurons were fixed with 4% paraformaldehyde for 10 min, blocked, and permeabilized in 0.1% Triton X-100 (Tx)-PBS with 10% fetal bovine serum for 15 minutes at room temperature. Then, neurons were incubated with the primary BIII-Tubulin antibody (1:500, ab78078, Abcam) in 0.1% Tx/10% FBS-PBS overnight at 4°C. Subsequently, wells were rinsed several times in PBS before applying the fluorochrome-conjugated secondary antibody (ThermoScientific) in 0.1% Tx/10% FBS-PBS for 1h at room temperature. Neurons were finally washed in PBS, and DAPI was added prior to the microscopic analysis. Acquired confocal images were quantified by measuring the BIII-Tubulin fluorescent integrated density and normalized by the number of DAPI-positive nuclei.

Tissue slices were rinsed in a phosphate buffered saline with 0.2% Triton (PBST) solution, non-specific binding was blocked with a 10% normal donkey serum (NDS)/PBST solution for 60 min at room temperature. Then tissue slices were incubated with the primary antibodies: Iba 1 (1:1000, WAKO), mouse anti-GFAP, (1:2000, Sigma) in a 1% NDS/PBST solution overnight at 4°C. Subsequently, slices were again rinsed several times in PBST before applying fluorochrome-conjugated secondary antibodies (Jackson Immunoresearch) in a 1% NDS/PBST solution with a 1:500 dilution for 60 min, effectively tagging any primary antibodies bound to protein from the first staining with a fluorescent label. Sections were finally washed in PBS and rinsed in water before mounting onto slides with Fluoromount G (Electron Microscopy Sciences) for microscopic analysis.

### Example 1.11. Oxygen Consumption

Bioenergetic profiles were measured using Seahorse XF96 Pro Analyzer (Agilent Technologies). Cells were plated in Seahorse XF96-Cell culture Microplates (Agilent) one day prior the assay. Cell density was calculated from each cell type according literature recommendation (https://cores.utah.edu/wp-content/uploads/2014/12/3.-Cell-line-density.xlsx). One hour before the assay, the medium was changed to Agilent Seahorse XF Media supplemented with 10mM D-glucose, 1mM pyruvate and 2mM L-glutamine, to a final volume of 180 μl per well. Cells were incubated in a 0% CO2 chamber at 37°C for 1h before the measure. For oxygen consumption rate (OCR) and ECAR experiments, we used Seahorse XF Cell Mito Stress Test Kit with different drug concentration depending on cell type. For mouse cortical neurons and astrocytes, we used 1.5 μM oligomycin, 2.5 μM FCCP and 1 μM rotenone/antimycin. For 143b cells and transmitochondrial cybrids, we used 1 μM oligomycin, 0,75 μM FCCP and 1 μM rotenone/antimycin. FAO was determined using the XF Cell Mito Stress Test with the XF Palmitate-BSA FAO Substrate, in the absence or presence of etomoxir, according to the manufacturer's instructions. Mouse cortical neurons and astrocytes were incubated overnight with 1mM D-glucose, 1mM L-glutamine, 0.5mM Carnitine, and 1% FBS. One hour before the experiment, the medium was changed to 2mM D-glucose and 0.5mM carnitine in a final volume of 135 μL per well. Fifteen minutes before running the assay, 15uL of 50uM etomoxir was added to selected wells. Finally, 30uL of Palmitate:BSA and BSA control were added just before running the assay. Additionally, we measured the indirect FAO respiration dependency by culturing astrocytes and neurons with etomoxir for 30 minutes prior to the Cell Mito Stress test to observe changes in basal respiration.

### Example 1.12. Metabolomics

1-5.0×10⁶ cells were harvested on dry ice with 1 mL pre-chilled 80% HPLC-grade methanol (Fluka Analytical). The cell mixture was incubated for 15 min on dry ice prior to centrifugation at 18,000 × g for 10 min at 4°C. The supernatant was retained, and the remaining cell pellet was resuspended in 800 μL chilled 80% methanol and centrifuged. Supernatant was combined with the previous retention and was lyophilized using a SpeedVac (Thermo Fisher). Lyophilized samples were resuspended in 20 μL ultrapure water and subjected to metabolomics profiling using the AB/SCIEX 5500 QTRAP triple quadrupole instrument. Data analysis was performed using the GiTools software. 13C-labeled glutamine and glucose was acquired in SIGMA (605166 and 389374).

### Example 1.13. PPP activity measurements

The determination of pentose-phosphate pathway (PPP) activity was conducted by assessing the difference between 14CO2 production from 1-14C-glucose, which decarboxylates through the 6-phosphogluconate dehydrogenase-catalyzed reaction, and that of 6-14C-glucose, which decarboxylates through the tricarboxylic acid cycle.

### Example 1.14. L- lactate release in neurons

L-lactate levels were measured in mouse cortical neurons using Lactate-Glo™ Assay (Promega, #J5021) following the original protocol.

### Example 1.15. ATP measurements in cells

ATP Assay Kit (Abcam, #ab83355) was used to analyze ATP levels. Briefly, Cells were seeded in M24 plates (neurons at 500,000 cells/mL and monolayer density for astrocytes), and for the ATP experiments, three wells per condition were combined. An alternative deproteinization protocol was applied to purify the samples, and a Colorimetric Assay was conducted as outlined in the original protocol. The measurements were obtained using the CLARIOstar Plus plate reader from BMG LABTECH.

### Example 1.16. ATP measurements in live neurons

ATP measurements was performed in primary co-cultures of cortical neurons and astrocytes from P1-2 wild-type rats of the Sprague-Dawley strain Crl:CD(SD), which are bred by Charles River Laboratories following the international genetic standard protocol (IGS). All experiments were carried out in strict accordance with the guidelines of the European Directive 2010/63/EU and the French Decree n° 2013- 118 concerning the protection of animals used for scientific purposes. Neurons were plate on poly- ornithine (Sigma-Aldrich, P3655) coated coverslips and maintained in culture media composed by MEM (Thermo Fisher Scientific, 51200-046), 20 mM Glucose (Sigma-Aldrich, G8270), 0.1 mg/mL transferrin (Sigma-Aldrich, 616420), 1% Glutamax (Thermo Fisher Scientific, 35050-038), 24 $\mu$g/mL insulin (Sigma-Aldrich, I6634), 5% FBS (Thermo Fisher Scientific, 10082-147), 2% N-21 (Bio-techne, AR008) and 4$\mu$M cytosine $\beta$-d-arabinofuranoside (Sigma-Aldrich, C6645). Cultures were incubated at 37°C in 95% air / 5% CO 2 humidified incubator.

Neurons were transfected at DIV 8 with ATeam1.03-nD/nA/pcDNA3 (gift from Takeharu Nagai (Addgene plasmid # 51958; http://n2t.net/addgene:51958 ; RRID:Addgene_51958)) and imaged at DIV 19. 24 h prior to imaging; 100 nM Piericidin A (Apex-Bio, C3535) was added to the culture. FRET-based ATP measurements were performed on an Axio Observer 7 inverted microscope (Zeiss) using a 63x Plan- Apochromat, 1.40 numerical aperture (NA), oil-immersion objective (Zeiss). Neurons were illuminated using a HXP 120V white light lamp. Fluorescence emission from ATeam was imaged using a FS 78 HE Dual camera; the exposure times were 250 ms for CFP and YFP images. Neurons were maintained during recording in Tyrode's solution (119 mM NaCl, 2.5 mM KCl, 2 mM CaCl 2, 2 mM MgCl 2, 30 mM glucose and HEPES 25mM pH 7.4) on a microscope at 37°C. Image analysis was performed using Image J Fiji 1.53t.

### Example 1.17. Animal care

C57BL/6J and NMRI-Foxn1$^{nu/nu}$ mice were purchased from Janvier Labs. Mice were housed in the Centro de Biología Molecular Severe Ochoa (CBMSO) Animal Facility and maintained under a 12-hours light/-dark cycle and on a standard chow diet. Animals lacking *Ndufs4* constitutively (*Ndufs4$^{-/-}$*, Ndufs4 KO) were originally generated by Richard D Palmiter, and all experiments involving these mice were conducted following the recommendations in the Guide for the Care and Use of Laboratory Animals and were approved by the Animal Care and Use Committee of the Universitat Autònoma de Barcelona (Barcelona, Spain). All animal procedures adhered to the guidelines outlined in the EU Directive 86/609/EEC and Recommendation 2007/526/EC, which pertain to the safeguarding of animals employed for scientific and experimental purposes. These regulations are enforced through Spanish law (RD 53/2013). All animal experiments were performed in accordance with national guidelines for housing and care of laboratory animals and the local ethics committee (CSIC) approved all animal studies (PROEX 123.5/21).

### Example 1.18. AAV9 Delivery

4 weeks-old mice were anesthetized with isoflurane (2.0 % induction, 0.75-0.25 % maintenance) and placed in a stereotaxic frame (Kopf Instruments). Ketoprofen analgesic solution (5 mg/kg s.c; Sanofi-aventis) and ocular protective gel (Viscotears®) were applied before stereotaxic procedure. Mice received intracranial AAV injections into the vestibular nucleus (VN) at the following coordinates: AP -5.88 mm, ML $\pm$ 1.0 mm, DV -4.25 mm from the Bregma, using the correction factor (Bregma - Lambda/4.21). A total of 1.0 $\mu$L of AAV9-Veh (AAV9-CMV-GFP) or AAV9_ME1 (AAV9-CMV-ME1-IRES-GFP) vector (0.7 $\times$ 10$^{12}$ viral genomes/mL; 0.5 $\mu$L per side) was injected into each hemisphere at a rate of 0.1 $\mu$L/min using a 5 $\mu$L Hamilton syringe with a 32G blunt needle. Following injection, the needle was kept in place for 7 min post-delivery to allow proper viral vector diffusion and, subsequently, removed at 1 mm/min. Viral particles were purchased from the Viral Vector Production Unit (UPV) (Universitat Autònoma de Barcelona-Vall d'Hebrón Institut de Recerca).

### Example 1.19. Xenografts experiments

Xenografts experiments were carried out in 4 weeks old female NMRI-Foxn1$^{nu/nu}$ mice that were subcutaneously injected with Colo320 cells. For each tumor, 2 million cells were resuspended in Matrigel Matrix: PBS solution (1:1) (Corning, #354234) and 100 uL of volume was injected in the flanks of each mouse. Tumors were measured every other day using digital vernier caliper until the volume reached 2 cm$^2$. At this point, they were sacrificed with $CO_2$ and posterior cervical disruption. Tumors were isolated and frozen in -80°C for future analysis. For volume calculation, we used the formula

$$V = \frac{(LxW^2)}{2}$$

in mm$^3$, were V = tumor volume, W = tumor width and L = tumor length.

## Example 1.20. Rotarod test

The rotarod test was used to monitor motor coordination. The task consisted of placing the animals on a rotating bar that accelerated from 4 rpm to 40 rpm (Harvard Apparatus, Holliston, MA, USA) at a lapse of 300 s until the animal fell to the ground. The latency time to fall from the rod and the speed (rpm) were also measured. Mice underwent five consecutive trials at P38-P39 (mid stage) and at P44-P45 (late stage), and the mean of the trials was calculated.

## Example 1.21. Open field test

The mice were placed in a methacrylate white box (56 [W], 36.5 [D], 31 [H] cm) and allowed to explore the apparatus freely for 30 min. The measured parameters include the total ambulation distance (horizontal activity) in the Arena and In Zone 1 during the test at P40-P41 (mid stage) and P50 (late stage). The activity of the mice was recorded through videography. The videos were analyzed using EthoVision XT tracking software (Noldus Information Technology bv., Wageningen, The Netherlands).

## Example 1.22. IACS treatment

To inhibit Mitochondrial Complex I *in vivo,* we employed IACS-010759 (Selleckchem, #S8731), a small-molecule inhibitor of complex I. IACS was dissolved in DMSO at a 10% concentration and then prepared for administration at 0.5% in Methyl cellulose (Sigma, #M0262). Each mouse received a dose of 10 mg/kg for five consecutive days via oral gavage.

## Example 1.23. Generation of *Drosophila* mutants

The following Drosophila stocks were used: Pgd$^{39}$ Zw$^{lo2a}$ (Bloomington Nr. 6033), UAS-ND49RNAi (Bloomington Nr. 57499), UASND51RNAi (Bloomington Nr. 52939) and rn-Gal4 (Bloomington Nr. 78345). To generate mutant flies for ND49 and ND51 UA-SRNAi were crossed with the rotund Gal4 driver (rn-Gal4). The flies were raised at 17°C until end of development. Double mutants with impaired mitochondrial and PPP activity were generated by crossing the stock Pgd$^{39}$ Zw$^{lo2a}$; UASND51RNAi or Pgd$^{39}$ Zw$^{lo2a}$; UASND51RNAi with rn-Gal4. All Drosophila strains were maintained in chambers conditioned with a relative humidity of 56% and regulated temperature of 17°C, in a medium of standart culture (15% yeast extract, 3,5% glucose, 2,5% flour, 0,6% agar, 0,7% propionic acid, 0,4% nipagin). Flies and pupae were pictured with a Leica MZ12 microscope with an installed Leica DFC490 camera and captured using the LAS (Leica Application Suite) V3.7 program.

## Example 1.24. Statistical Analysis

Data are shown as the mean $\pm$ SEM. GraphPad Prism 10 software was used for statistical analyses. All statistics are described in figure legends. In general, for two experimental comparisons, a two-tailed unpaired Student's t-test was used. For multiple comparisons, one-way ANOVA or two-way ANOVA was applied. Three replicates per treatment were chosen as an initial sample size unless otherwise stated in the figure legends. All western blot analysis were repeated at least three times.

## Example 2. Results

## Example 2.1. PPP inhibition is synthetic lethal with mitochondrial dysfunction

Altered glucose metabolism is a hallmark of human disorders associated with mitochondrial dysfunction. Using genetic and pharmacological approaches to inhibit the ETC, we noticed that respiration-deficient cells were highly sensitive to glucose withdrawal. This is agreement with previous *in vitro* and *in vivo* observations showing that enhanced glucose uptake and its further catabolism through glycolysis is a well-known consequence of ETC inhibition. It has long been assumed that glucose entering ETC-deficient cells is mostly channeled through the glycolytic pathway as an adaptation to switch ATP production from OXPHOS to glycolysis. However, it is known that besides glycolysis, glucose molecules are important to sustain other metabolic pathways such as pentose phosphate pathway, hexosamine pathway and serine biosynthesis pathway. Additionally, glucose-derive pyruvate may enter the mitochondria to fuel anabolic and catabolic reactions **(Figure 1A).**

To investigate glucose-dependent alternative metabolic pathways that become essential upon ETC inhibition, we generated through CRISPR/Cas9 technology, knock-out (KO) 143B cell lines for each of the following rate-limiting enzymes and transporters. We selected glucose 6 phosphate dehydrogenase (G6PD) for the PPP, Glutamine-Fructose-6-Phosphate Transaminase 1 (GFPT1) for the hexosamine pathway, Phosphoglycerate Dehydrogenase (PHGDH) for the serine biosynthesis pathway and Mitochondrial Pyruvate Carrier 1 (MPC1) to prevent pyruvate entry into the mitochondria. These KO and Control cells were treated with two specific ETC inhibitors (Piericidin A that targets Complex I and Antimycin A that targets Complex III) and cell viability was assessed after 3 days of treatment. Surprisingly, G6PD depletion render cells sensitive to ETC inhibition while KO cells for GFPT1, PHGDH or MPC1 showed no differences in survival or proliferation **(Figure 1B).** These results were confirmed using specific small molecule inhibitors for each pathway in human cybrid cells carrying a mutation in the Complex I subunit ND2 **(Figure 1C).** Further, cell proliferation was markedly reduced in ND2, CytB and Cox2 cybrid cells treated with selective cell penetrant inhibitors of PPP **(Figure 1D).** Fascinated by these results, we wondered whether this phenotype was also reproduced *in vivo.* We took advantage of the fruit fly *Drosophila melanogaster* model, as it is cost- and time-efficient and recapitulate a wide range of phenotypes seen in patients with metabolic and mitochondrial disorders. We obtained flies carrying mutations in Zw and Pgd genes (named: Pgd[39] Zw[lo2a]), corresponding to human orthologs G6PD and 6PGD respectively. We also obtained flies carrying an siRNA targeting ND49 (ND49-RNAi) or ND51 (ND5-RNAi), both structural subunits of the mitochondrial complex I, corresponding to human orthologs NDUFS2 and NDUFV1 respectively. We then crossed these flies to generate double mutants with impaired mitochondrial and PPP activity. Similar to WT, Pgd[39]Zw[lo2a] flies with defective PPP developed normally and did not display any overt phenotype. Flies with downregulation of complex I subunits ND49 and ND51 by RNAi had difficulties to hutch and the majority remained in the pupae stage while only ~ 40 to 5% developed normally. Strikingly, and in line with our *in vitro* data, double mutant flies with impaired mitochondrial and PPP activity exhibited a quite dramatic phenotype. As shown in **Figure 1E,** these mutants were not able to reach the larval stage and died prematurely as undeveloped pupae. Overall, this data concludes the existence of an unexpected synthetic lethality between PPP deficiency and mitochondrial dysfunction that is conserved at the organismal level.

Increased lactate secretion is considered a good indicator of enhanced glycolytic rates, which is a common feature of ETC-inhibited cells as they switch from OXPHOS to glycolysis to maintain ATP levels. Since we uncovered that PPP becomes essential in cells with disrupted ETC we sought to determine if PPP activity is also adaptably upregulated. We culture 143B cells in the presence of Piericidin for 24 h and PPP activity and lactate release, as a proxy of glycolysis, were measured. As expected, lactate detection in the media was higher in CI-inhibited cells. Strikingly, we also observed a significant increase in PPP activity in Piericidin treated cells. This phenotype was also conserved *in vivo* as we detected elevated levels of PPP metabolites in the brain of Ndufs4 KO mice, a model of mitochondrial disease resembling human Leigh syndrome, which overall indicates that when ETC function is compromised, glucose flux is augmented not only towards glycolysis but also through PPP. Next, we wonder if in this context, glucose can be overused by glycolysis and therefore diverted away from the PPP. Diseases associated with impaired mitochondrial function negatively impact high energy demanding tissues such as the brain. As such, neurons that are highly sensitive to ETC inhibition also rely strongly on glucose metabolism. Thus, we used mouse cortical neurons to determine the rates of glycolysis and PPP as glucose levels become limiting. Control and Piericidin treated neurons where cultured in media containing supraphysiological (25 mM), physiological (5 mM) or subphysiological concentrations of glucose (2,5 mM). Similar to 143B cells, we observed a significantly upregulation of both lactate and PPP activity in neurons cultured in 25 mM glucose. However limited glucose availability caused a prominent drop-in PPP activity while preserving the levels of lactate. Curiously, a trend towards reduced PPP activity and increased lactated secretion was observed in 5 mM cultured neurons although it did not reach statistical significance **(Figure 1F).** Decreased PPP activity under low glucose (2,5 mM) correlated with a rapid decline in NADPH/NADP+ levels in CI-inhibited cells. Conversely, glucose availability had no effect on ATP levels at this timepoint. Hence, our interpretation is that when glucose is scarce, neurons decide to maintain glycolysis at the expense of PPP, likely to avoid a catastrophic decline in intracellular ATP levels.

## Example 2.2. Increased expression of ME1 diminish synthetic lethality between PPP and OXPHOS

Next, we conducted time-course experiments to gain further knowledge on the metabolic determinants underlying the observed synthetic lethality between PPP and OXPHOS. 143B cells were treated either with Piericidin or G6PDi (selective inhibitor of G6PD) alone or the combination of both and cell proliferation was quantified over the course of 96 h. Abrogation of CI or PPP separately did not cause a substantial decline in cellular fitness. Conversely, dual inhibition produced a marked reduction in cell survival beyond 48 h of treatment that was even more accentuated at 96 h **(Figure 2A).** Since 48 h indicated the onset of the phenotype, we conducted metabolomic analyses at this particular timepoint to generate a wider, unbiased view of the metabolic rearrangements that occur when the ETC and PPP are compromised. Blocking CI, PPP or both resulted in massive alteration of many intracellular metabolites **(Figure 2B).** We observed that CI or PPP inhibition led to expected changes in specific metabolites, for instance accumulation of NADH or depletion of 6-phospho-d-gluconate respectively. However, the combination of both drugs created a specific metabolic profile. We focused on the top metabolites that were selectively dysregulated in the condition where both CI and PPP were inhibited. Interestingly, dihydroxyacetone phosphate and sedoheptulose-7-phosphate were markedly downregulated in this scenario which might reflect a substantial redirection of glucose molecules towards glycolysis at expenses of completely shutting down the non-oxidative phase of pentose phosphate pathway **(Figure 2C and** D). Another metabolite that followed a similar trend was NADPH

**(Figure 2E).** Piericidin or G6PDi treatment alone did not exert significant changes in the intracellular levels of NADPH, suggesting that when PPP is nonfunctional, mitochondrial activity is enough to maintain NADPH homeostasis and vice versa. However, the combination of both led to a significant reduction in NADPH at 48 h, that was further aggravated at 72 h **(Figure 2F)** and correlated with increased oxidative stress at this timepoints **(Figure 2G)**. Of note, the cell death phenotype could be rescued by supplementing cells with GSH **(Figure 2SD)**. This suggests that NADPH homeostasis and management of oxidative stress rely on active mitochondrial function when PPP activity becomes compromised.

Because NADPH metabolism is naturally linked to oxidative stress, a well-known pathogenic event commonly observed in diseases associated with mitochondrial dysfunction, we were interested in discovering NADPH-generating systems that could alleviate this phenotype. Regeneration of cytosolic NADPH from NADP+ is controlled by three well-validated routes: ME1, IDH1 and the oxidative pentose-phosphate pathway (oxPPP), in which NADPH is produced by both G6PD and 6-phosphogluconate dehydrogenase (PGD) **(Figure 2SE)**. Thus, we focued on ME1 and IDH1 as tools to potentially rise NADPH levels after inhibition of oxPPP and ETC. We generated 143B cells stably overexpressing either ME1 or IDH1 and performed survival analysis on these cells **(Figure 2SF)**. Interestingly, ectopic expression of ME1 completely restored cell survival while forced expression of IDH1 only achieved a milder phenotype **(Figure 2H)**. These results were consistent as ME1 expressing cells showed less oxidative stress than those expressing IDH1 **(Figure 2SG)**. At this point, we were intrigued about the disappointing effects of IDH1 expression compared to ME1. Substrates of ME1 and IDH1 are malate and isocitrate respectively, which are intermediate metabolites associated with the TCA cycle. To gain further insight into how ETC inhibition could elicit changes in ME1 and IDH1 substrates, we profiled TCA cycle metabolites using LC-MS based metabolomics analysis. Surprisingly, malate and isocitrate emerged as the most dysregulated metabolites in the context of ETC disruption. While CI inhibition caused a modest increase in intracellular malate, fumarate and OAA, levels of succinate and most noticeably isocitrate sank extremely **(Figure 2I)**. This could explain why forced expression of IDH1 was not enough to restore cell survival and normalize ROS levels in **Figure 2H** and **Figure 2SG**. We therefore hypothesized that boosting intracellular levels of isocitrate would be beneficial to IDH1-expressing cells. Indeed, supplementation with trimethyl-citrate, a cell permeable citrate that can be converted intracellularly to isocitrate, protected 143B cells from oxidative stress in an IDH-dependent manner **(Figure 2J)**. Altogether, these results indicate that ME1 expression and activity are limiting factors determining cell survival when the PPP and ETC are compromised.

**Example 2.3. Rewiring of cellular metabolism through PC and ACLY support Malate production after CI inhibition**

Encouraged by the beneficial effects of ME1 and because ME1-dependent NADPH production is linked to intracellular malate levels, we sought to investigate the metabolic pathways and nutrients that maintain malate production upon disruption of the ETC. It is firmly believed that in cells and tissues with defective ETC function, elevated NADH/NAD+ ratio inhibits the Pyruvate dehydrogenase complex (PDC), suppressing pyruvate entry into the mitochondria and forcing its channelling towards the Lactate Dehydrogenase (LDH) in the cytosol. In contrast, mitochondrial utilization of glutamine has been shown to be elevated in OXPHOS-deficient cells'. Hence, we hypothesize that upon ETC inhibition, malate levels could be maintained through altered glutamine metabolism. To investigate the metabolic routes that ETC-inhibited cells used to generate glutamine-derived malate, we performed an MS-based stable isotope tracing study. DMSO and Piericidin treated 143B cells were cultured in glucose-DMEM supplemented with 2 mM of uniformly labeled [$^{13}$C]glutamine, and the incorporation of $^{13}$C into the TCA cycle and related metabolic intermediates was quantified. Oxidative metabolism of [U-$^{13}$C]glutamine would generate M+4 isotopologues of oxaloacetate (OAA) and malate, while reductive carboxylation due to ETC inhibition (TCA cycle running in a counterclockwise direction) would produce M+3 forms of OAA and malate. DMSO-treated 143B cells used glutamine as the major anaplerotic precursor, resulting in a large amount of malate M+4. As expected, the oxidation of glutamine via the TCA cycle in CI-inhibited cells was severely diminished with a 5-fold decreased in M+4 labeled malate. Nevertheless, these cells produced citrate M+5 through reductive carboxylation of glutamine-derived a-ketoglutarate. This reaction involves addition of an unlabeled carbon by IDH2 acting in reverse relative to the canonical oxidative TCA cycle. Malate M+3 also rose more than a ~2-fold in CI-inhibited cells and was likely formed after cleavage of citrate M+5 by ATP-citrate lyase (ACLY) since this induction was blunted in ACLY KO cells. Thus, cells with defective ETC use glutamine-dependent reductive carboxylation rather than oxidative metabolism as the major pathway of malate production. Strikingly, Piericidin treatment did not translate into increased cell death or impaired cell proliferation in ACLY KO cells potentially indicating that further adaptive mechanisms were ensuring malate production in CI-inhibited ACLY KO cells. We hypothesized that an alternative mechanism could involve pyruvate entry into the mitochondria where it can be carboxylated to OAA by the pyruvate carboxylase (PC). In fact, this can be beneficial to cells with defective ETC because it diminishes the need to recycle reduced electron carriers generated by the TCA cycle compared to glutamine oxidation. To examine the contribution of PC to malate formation in the context of ETC dysfunction, control and PC KO cells were cultured with uniformly labeled [$^{13}$C]glucose and treated either with DMSO or Piericidin. In this labeling scheme, oxidative metabolism of glucose-derived pyruvate would give rise to TCA cycle intermediates with M+2 labeling as $CO_2$ is removed during conversion of pyruvate to acetyl-CoA by pyruvate dehydrogenase (PDH). By contrast, carbon labels are retained if pyruvate is carboxylated by PC and would generate M+3 intermediates (**Figure 3A**). Piericidin treatment clearly reduced PDH-dependent labeling of aspartate and malate both in control and PC KO cells, consistent with PDH activity being hindered by the resulted increased in NADH/NAD+ ratio. However, CI-inhibited cells displayed a marked induction in PC-mediated labeling of TCA cycle intermediates, particularly aspartate and malate (**Figure 3A**). Malate and aspartate M+3/M+2 ratios, which reflect the relative contribution of PC versus PDH to metabolite labeling, were ~7-fold and ~10-fold higher respectively, in cells treated with Piericidin (**Figure 3B and C**). Together, these data demonstrate that, in cells with ETC dysfunction, pyruvate is not only converted to lactate, but also imported inside the mitochondria where it contributes to malate formation in a PC-dependent manner. In this regard, it is not surprising that PC activity is favoured over PDH when the ETC is defective as it is not regulated by NADH levels.

Surprisingly, just like ACLY KO cells, ablation of PC did not sensitize to ETC inhibition and PC KO cells proliferated normally after Piericidin treatment (**Figure 3D**). To assess the relative contribution of PC and ACLY to cell death phenotype in ETC deficient cells upon PPP inhibition, we treated PC KO and ACLY KO cells with Piericidin, G6PDi or both. A massive reduction in cell viability was observed as early as 48 h in both PC KO and ACLY KO cells after dual inhibition (**Figure 3E),** which correlated with a significant decrease in the intracellular levels of malate (**Figure 3F**). A plausible explanation for the lack of phenotype in both PC and ACLY KO cells could be the upregulation of compensatory mechanisms whereby PC depletion would cause an enhanced rerouting of glutamine through ACLY and vice versa. To test this hypothesis we performed tracing experiments using [U-$^{13}$C]glutamine in PC KO cells. Consistent with our previous experiments, Piericidin treatment caused a reduction of malate M+4 while increasing the labeling of malate M+3. Notably, cells with PC depletion exhibited a substantial increase in malate M+3 compared to control cells, suggesting that when glucose-derived malate production from PC is impaired, cells respond by increasing glutamine-derived malate production through ACLY (**Figure 3G).** To further validate this theory, we treated ACLY KO cells with a specific inhibitor of glutaminase 1 (GLS1) to block mitochondrial glutamine metabolism and PC KO cells with a specific inhibitor of the mitochondrial pyruvate carrier (MPC) to prevent glucose-derive pyruvate entry into the mitochondria. Upon CI inhibition, a significant decrease in cell viability was evident in PC KO and ACLY KO cells that had been treated with the GLS1 inhibitor or MPC inhibitor respectively. Finally, double ablation of PC and ACLY, in contrast to single KO cells, sensitize cells to ETC inhibition (**Figure 3H**) with a parallel decrease in intracellular malate levels (**Figure 3I**). Taken together, these results indicate the TCA cycle is adaptively rewired after ETC inhibition, to ensure continuous production of malate which is further metabolized by ME1 to generate NADPH. We found that this compensatory mechanism is characterized by a high degree of nutrient flexibility where both glucose and glutamine, through different metabolic routes, can contribute to malate production.

## Example 2.4. ME1 expression levels dictates cancer vulnerability to OXPHOS inhibitors

To investigate whether this was a general phenomenon conserved across multiple cellular models we conducted in silico analyses using proteomics data of 375 cell lines from diverse lineages in the Cancer Cell Line Encyclopedia (CCLE). Cells were categorized according to their ME1 protein expression levels and we were able to obtain cells corresponding to the top and bottom 5% for further analysis (**Figure 4A** and **4SA**). Based on our previous data, we speculate that cells with high ME1 expression will be insensitive to combine inhibition of ETC and PPP while cells expressing low levels of ME1 will be highly sensitive. In agreement with our hypothesis, HCC15, HYSE410, CAKI1 and J82 cell lines with high ME1 levels proliferated normal when treated with Piericidin and G6PDi. In contrast, MDAMB453, Colo320, NCIH1703, OCI-Ly3 and RCH-ACV which represent cell lines with very low levels of ME1 were extremely sensitive and died after 4 days of treatment (**Figure 4B).** We noticed that high ME1 expressing cells were able to maintain a normal NADPH/NADP+ ratio whereas Piericidin+G6PDi treatment in cells with low levels of ME1 caused a marked decrease in the levels of NADPH (**Figure 4C).** Next, we wanted to corroborate that this phenotype was solely caused by the differential expression of ME1 in these cell lines. To this end, ME1 was either depleted in HCC15 and J82 high ME1 expressing cells or overexpressed in Colo320 and MDAMB453 low ME1 expressing cells. Piericidin+G6PDi treatment negatively impacted cell viability of HCC15 and J82 KO cells (**Figure 4D**) but had little effect in Colo320 and MDAMB453 cells overexpressing ME1 (**Figure 4E).** This data suggests that the levels of ME1 can modulate synthetic lethality between PPP and OXPHOS in multiple cell lines.

We propose that sensitivity to OXPHOS inhibitors can be, at least in part, mediated by combined expression of the NADPH-producing enzymes G6PD and ME1. To test this hypothesis *in vivo,* we correlated protein expression of G6PD and ME1 for hundreds of cell lines. This allowed us to stratify these cells into two well-defined categories; ETC-resistant correspond to all the cell lines predicted to be insensitive to CI inhibition, whereas ETC-sensitive represent cells whose fitness greatly depend on CI activity. **(Figure 4F).** Among the top ETC-sensitive cell lines, we selected Colo320, a colorectal adenocarcinoma human cell line that has been shown to form solid tumors in xenograft models. Western blot analysis confirmed low levels of both ME1 and G6PD in this cell line. It is widely known that cancer cells within solid tumors are usually poorly vascularized and have restricted access to nutrients such as glucose. Thus, the combination of low glucose availability and limited PPP activity (due to low levels of G6PD) would render Colo320 cells sensitive to the inhibition of CI *in vivo.* Colo320 cells were injected subcutaneously into the flank of nude mice that were either treated with vehicle or IACS-010759, a clinical-grade small-molecule inhibitor of complex I. Tumor progression analysis confirmed that IACS administration prominently reduced tumor size. However, this effect was completely lost in Colo320 cells ectopically expressing ME1 **(Figure 4G).** This data indicates that *in vivo,* ME1 expression is mayor factor determining the susceptibility to OXPHOS inhibition when PPP activity is limited.

### Example 2.5. Differential sensitivity to mitochondrial complex I inhibition between astrocytes and neurons

ETC defects occurring from mitochondrial disease mutations compromise cellular fitness and survival. As a consequence of this biochemical failure, cellular damage appears particularly in cells of high energy demanding tissues such as brain. Interestingly, the brain is a highly heterogeneous organ composed of several types of cells that are not equally sensitive to mitochondrial dysfunction. In alignment with prior studies demonstrating that ETC inhibition does not compromise the survival of astrocytes both *in vitro* and *in vivo,* our observations revealed that Piericidin treatment in mouse primary astrocytes had no discernible impact on their morphology, proliferation, or overall viability. In contrast, primary neurons subjected to CI inhibition exhibited significant morphological alterations, marked by extensive axon degeneration, lower capacitance and diminished excitability response. Intriguingly, this change in morphology did not correspond to an increase in neuronal death at these timepoints. This is consistent with recent findings showing that the deletion of the CI subunit Ndufs2 in dopaminergic neurons does not induce neuronal death. Instead, it leads to a gradual axonal dysfunction and loss of cell identity. Thus, a fundamental question that remains to be addressed is the identification of the molecular underpinnings that underlie the differential sensitivity to perturbations in the ETC among these brain cell types. It is widely accepted that neurons exhibit a prominent oxidative metabolism characterized by high levels of mitochondrial activity whereas astrocytes perform aerobic glycolysis with lactate extrusion into the extracellular space as the endpoint and in this way are more protected from mitochondrial dysfunction. We considered that this explanation is rather simplistic and poorly sustained by empirical data. In fact, our seahorse experiments measuring of oxygen consumption rate (OCR) in astrocytes and neurons demonstrated that both cell types are able to perform mitochondrial dependent oxidative metabolism. While neurons displayed a greater capacity to sustain oxygen consumption when glucose was the main substrate (**Figure 5A** and **S5F**), astrocytes exceeded neurons in their ability to oxidize fatty acids (**Figure 5B, C** and **S5G**). These results proved that both cell types rely on oxidative mitochondrial metabolism, but in a nutrient dependent manner and suggest that low ETC activity in astrocytes cannot be the reason why these cells are protected against OXPHOS inhibitors.

At this point, we were curious if any of the NADPH generating enzymes and associated proteins were somehow differentially expressed between astrocytes and neurons. Protein expression analysis revealed that PC and ME1 were selectively expressed in astrocytes compared to neurons whereas IDH1 and ACLY showed minimal differences **(Figure 5D).** Furthermore, stable-isotope tracing metabolomics experiments confirmed that PC activity was more than 2-fold higher in astrocytes versus neurons (**Figure 5E** and **S5H**). Of note, we validated that astrocytes had increased capacity to transform glucose into lactate. Next, we investigated whether CI inhibition differentially impact bioenergetics and redox homeostasis in these brain cell types. Piericidin treatment did not cause any alteration in ATP or NADPH levels in astrocytes **(Figure 5F).** Strikingly, CI-inhibited neurons showed a marked drop in NADPH levels while ATP levels were maintained, at least at these timepoints **(Figure 5G).** Surprised by the ability of neurons to sustain ATP levels even when the ETC is not functioning, we conducted a more refined experiment using a genetically encoded ATP biosensors targeted to either the soma or the axons. Again, Piericidin treatment did not altered ATP in any of these compartments, suggesting that acute inhibition of CI is well tolerated by neurons and astrocytes most likely by adaptive upregulation of glycolytic-derived ATP.

Intrigued by the natural ability of astrocytes to maintain intracellular levels of NADPH, we sought to determine if this mechanism was mediated by their higher expression of PC and ME1. Silencing PC and ME1 increased their sensibility towards CI inhibition. PC and ME1 knockdown (KD) astrocytes proliferated less in the presence of Piericidin compared to WT controls (**Figure 5H** and **S5J**) and this phenotype correlated with reduced levels of NADPH **Figure 5I**. Next, we aimed to enhance the levels of ME1 as a mean to restore the functionality of CI-inhibited neurons. Lentiviral-mediated overexpression of ME1 in primary neurons successfully restored NADPH levels (**Figure 5J** and **S5K**) and improved both capacitance and excitability (**Figure 5K** and **S5L**).

Overall, these results suggest that astrocytes exhibit a functional ETC and function as oxidative cells. However, in contrast to neurons, their primary choice for oxidation leans towards fatty acids over glucose-derived pyruvate. Moreover, it appears that astrocytes possess a natural defense mechanism against insults that inhibit the ETC. This protection is attributed to their higher expression levels of PC and ME1 enzymes, which enable them to uphold malate levels and prevent disruptions in NADPH production.

### Example 2.6. Increasing brain levels of ME1 mitigates neuroinflammation and enhances motor coordination in Ndufs4$^{-/-}$ mice

Having confirmed the crucial role of ME1 in orchestrating metabolic adaptations necessary for preserving cellular fitness when CI is impaired, we aimed to assess the consequences of augmenting ME1 *in vivo,* specifically within the context of CI dysfunction. Consequently, we conducted a study to investigate the therapeutic potential of boosting ME1 expression in an *in vivo* model of CI deficiency, employing Ndufs4 KO mice. These mice exhibit progressive fatal neuroinflammation and neurodegeneration, mirroring the features observed in human Leigh syndrome. We generated an Adeno-associated virus of serotype 9 (AAV9), with tropism to the central nervous system, as a vector to deliver and enhance ME1 levels within the brain. We conducted stereotaxic injection of AAV9-control and AAV9-ME1 viral particles into the vestibular nuclei of 4-week-old Ndufs4 KO mice, and disease progression was monitored over time **(Figure 6A).** The overexpression of ME1 failed to reverse the body weight loss in Ndufs4 KO mice and did not extend their survival (**Figure 6B** and **6SA**). We proceeded to investigate the brain pathology and motor function in age-matched injected mice. Consistent with previous reports, Ndufs4 KO mice exhibited a progressive development of ataxia and locomotor deficits.

Notably, the augmentation of ME1 levels did not affect the locomotion phenotype but led to a substantial preservation of motor function and coordination **(Figure** C and S6B). This phenotype was associated with a marked reduction in the levels of neuroinflammation, as indicated by a significant decrease in the number of Iba1-positive microglia in the vestibular nuclei of AAV9-ME1 injected mice **(Figure** 6D). Thus, heightening ME1 levels proves to be beneficial in a preclinical mouse model of Leigh syndrome with impaired CI activity.

Taken together, our findings have revealed an adaptive mechanism where cells experiencing ETC inhibition undergo a transition to a non-canonical TCA cycle to uphold malate levels. Subsequently, this malate is harnessed by ME1 to generate NADPH, a process that becomes particularly relevant in cases where the pentose phosphate pathway (PPP), a primary source of NADPH, is compromised **(Figure 7).** These results provide fundamental insights into the molecular and metabolic factors that contribute to the distinct susceptibility of cell types and tissues to mitochondrial dysfunction and hold profound implications for enhancing our understanding of mitochondrial diseases.

**Claims**

1. NADPH for use in a method for the treatment of mitochondrial diseases.

2. NADPH for use, according to claim 1, in a method for the treatment of neurodegenerative mitochondrial diseases.

3. NADPH for use, according to any of the previous claims, wherein the method comprises promoting the production of NADPH by increasing the expression and/or activity of enzymes that produce NADPH.

4. NADPH for use, according to any of the previous claims, wherein the method comprises promoting the production of NADPH by increasing the expression and/or activity of enzymes that produce NADPH, preferably by means of the administration of a vector to deliver and enhance the expression and/or activity of enzymes that produce NADPH in the brain or by means of the administration of molecule able to enhance the expression and/or activity of enzymes that produce NADPH in the brain.

5. NADPH for use, according to any of the previous claims, wherein the vector is an adeno-associated virus with tropism to the central nervous system.

6. NADPH for use, according to any of the previous claims, wherein the enzyme that produce NADPH is selected from malic enzyme, isocitrate dehydrogenase 1, or glucose 6 phosphate dehydrogenase.

7. *In vitro* method for the diagnosis and/or prognosis of mitochondrial diseases which comprises assessing the level of NADPH, or of the expression level and/or activity of enzymes that produce NADPH, in a biological sample obtained from the subject.

8. *In vitro* use of NADPH, or of an enzyme that produce NADPH, or of a kit comprising reagents for the identification of NADPH, or of an enzyme that produce NADPH, for the diagnosis and/or prognosis of mitochondrial diseases.

9. *In vitro* method, or *in vitro* use, according to any of the claims 7 or 8, for the diagnosis and/or prognosis of neurodegenerative mitochondrial diseases.

10. *In vitro* method, or *in vitro* use, according to any of the claims 7 to 9, which comprises assessing the level of expression and/or activity of enzymes that produce NADPH, in a biological sample obtained from the subject, selected from malic enzyme 1, isocitrate dehydrogenase 1, or glucose 6 phosphate dehydrogenase.

11. *In vitro* method, or *in vitro* use, according to any of the claims 7 to 10, which comprises assessing the level of NADPH or the expression level and/or activity of enzymes that produce NADPH, in a biological sample obtained from the subject, wherein a lower level of NADPH or a lower level of expression and/or activity of enzymes that produce NADPH, as compared to a pre-established threshold level determined in control subjects who are not suffering from neurodegenerative mitochondrial diseases, is an indication that the subject has a pathology associated with mitochondrial dysfunction or a mitochondrial disease or has a poor prognosis.

12. *In vitro* method, or *in vitro* use, according to any of the claims 7 to 11, wherein the biological sample is selected from tissue, blood, serum, plasma or cerebrospinal fluid.

**Figure 1**

**Figure 1 (cont.)**

**Figure 1 (cont.)**

# Figure 1 (cont.)

**Figure 2**

**Figure 2 (cont.)**

**Figure 2 (cont.)**

**Figure 2 (cont.)**

**Figure 2 (cont.)**

**Figure 2 (cont.)**

**Figure 3**

**Figure 3 (cont.)**

B

DMSO □ Piericidin

Malate M+3/M+2

C Aspartate M+3/M+2

**Figure 3 (cont.)**

**Figure 3 (cont.)**

F

Malate levels (relative to DMSO)

P<0.001
n.s

| Pier | − | + | − | + |
| G6PDi | − | − | + | + |

○ DMSO  ▪ Piericidin

**[U-¹³C]Glutamine ⟶ Malate**

G

Malate M+4

Fraction of total metabolite

sgCtrl    sgPC

Malate M+3

Fraction of total metabolite

P=0.0022

sgCtrl    sgPC

**Figure 3 (cont.)**

Figure 4

EP 4 653 001 A1

**Figure 4 (cont.)**

**Figure 4 (cont.)**

**Figure 4 (cont.)**

**Figure 4 (cont.)**

**Figure 5**

**Figure 5 (cont.)**

**Figure 5 (cont.)**

E

## [U-$^{13}$C]Glc → Malate

F

## Astrocytes

**Figure 5 (cont.)**

**Figure 5 (cont.)**

**Figure 5 (cont.)**

**Figure 6**

**Figure 6 (cont.)**

**Figure 6 (cont.)**

**Figure 7**

Europäisches
Patentamt

European
Patent Office

Office européen
des brevets

# EUROPEAN SEARCH REPORT

**Application Number**

EP 24 38 2547

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CONNOLLY NIAMH M C ET AL: "Guidelines on experimental methods to assess mitochondrial dysfunction in cellular models of neurodegenerative diseases", CELL DEATH & DIFFERENTIATION, NATURE PUBLISHING GROUP, GB, vol. 25, no. 3, 11 December 2017 (2017-12-11), pages 542-572, XP037433895, ISSN: 1350-9047, DOI: 10.1038/S41418-017-0020-4 [retrieved on 2017-12-11] * page 554 * * page 566 * ----- | 7-9,11, 12 | INV. A61K31/7084 A61K48/00 A61P25/00 A61P25/28 C12N15/86 C12Q1/32 G01N33/50 |
| X | WO 2022/008802 A1 (HELSINGIN YLIOPISTO [FI]) 13 January 2022 (2022-01-13) * page 32 - page 36; claims 1,3,28,29,57,61,63-65,68 * ----- | 7-12 | |
| X | WO 2015/069872 A2 (UNIV ILLINOIS [US]; LU YI [US]; XIANG YU [US]; ZHANG JINGJING [US]) 14 May 2015 (2015-05-14) * page 29 - page 34; claim 9 * ----- | 7-12 | **TECHNICAL FIELDS SEARCHED (IPC)** A61K G01N C07K |
| X | ZHOU JING-SI ET AL: "NADPH ameliorates MPTP-induced dopaminergic neurodegeneration through inhibiting p38MAPK activation", ACTA PHARMACOLOGICA SINICA, NATURE PUBLISHING GROUP, GB, vol. 40, no. 2, 16 May 2018 (2018-05-16), pages 180-191, XP036672672, ISSN: 1671-4083, DOI: 10.1038/S41401-018-0003-0 [retrieved on 2018-05-16] * abstract * * see Results * ----- -/-- | 1,2 | A61P C12N C12Q |

The present search report has been drawn up for all claims

| Place of search | Date of completion of the search | Examiner |
|---|---|---|
| Munich | 8 November 2024 | Pacreu Largo, Marta |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 444 053 A (BIRKMAYER JOERG G D [AT]) 22 August 1995 (1995-08-22) * claims * | 1,2 | |
| X | WO 2010/121010 A2 (HARVARD COLLEGE [US]; CEPKO CONSTANCE L [US]; PUNZO CLAUDIO [US]) 21 October 2010 (2010-10-21) | 3,4 | |
| Y | * claims 2,6,7,10,19,20, * | 3-6 | |
| X | BALSA EDUARDO ET AL: "Defective NADPH production in mitochondrial disease complex I causes inflammation and cell death", NATURE COMMUNICATIONS, vol. 11, no. 1, 1 June 2020 (2020-06-01), XP093221790, UK ISSN: 2041-1723, DOI: 10.1038/s41467-020-16423-1 Retrieved from the Internet: URL:https://www.nature.com/articles/s41467-020-16423-1> * page 8 - page 9; figure 6 * | 3-6 | |
| | | | TECHNICAL FIELDS SEARCHED (IPC) |
| Y | WO 02/08405 A1 (TG BIOTECH INC [KR]; HUH TAE LIN [KR] ET AL.) 31 January 2002 (2002-01-31) * claims * | 3-6 | |

-/--

The present search report has been drawn up for all claims

| Place of search | Date of completion of the search | Examiner |
|---|---|---|
| Munich | 8 November 2024 | Pacreu Largo, Marta |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

**Application Number**

EP 24 38 2547

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | GORMAN GRÁINNE S. ET AL: "Mitochondrial diseases", NATURE REVIEWS. DISEASE PRIMERS, vol. 2, no. 1, 20 October 2016 (2016-10-20), XP093222162, GB ISSN: 2056-676X, DOI: 10.1038/nrdp.2016.80 Retrieved from the Internet: URL:https://www.nature.com/articles/nrdp2016080> * abstract * | 1-6 | |
| A | KOJU NIRMALA ET AL: "Reduced nicotinamide adenine dinucleotide phosphate in redox balance and diseases: a friend or foe?", ACTA PHARMACOLOGICA SINICA, NATURE PUBLISHING GROUP, GB, vol. 43, no. 8, 11 January 2022 (2022-01-11), pages 1889-1904, XP037923611, ISSN: 1671-4083, DOI: 10.1038/S41401-021-00838-7 [retrieved on 2022-01-11] * the whole document * | 1-12 | |
| A | WO 2007/035496 A1 (EDISON PHARMACEUTICALS INC [US]; MILLER GUY M [US] ET AL.) 29 March 2007 (2007-03-29) * claim 9 * | 1-6 | TECHNICAL FIELDS SEARCHED (IPC) |

The present search report has been drawn up for all claims

| Place of search | Date of completion of the search | Examiner |
|---|---|---|
| Munich | 8 November 2024 | Pacreu Largo, Marta |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 .............................................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 38 2547

This annex lists the patent family members relating to the patent documents cited in the above-mentioned European search report.
The members are as contained in the European Patent Office EDP file on
The European Patent Office is in no way liable for these particulars which are merely given for the purpose of information.

08-11-2024

| Patent document cited in search report | | | Publication date | Patent family member(s) | | | Publication date |
|---|---|---|---|---|---|---|---|
| WO 2022008802 | A1 | | 13-01-2022 | AU | 2021303586 | A1 | 09-02-2023 |
| | | | | CN | 116249904 | A | 09-06-2023 |
| | | | | EP | 4179329 | A1 | 17-05-2023 |
| | | | | JP | 2023532786 | A | 31-07-2023 |
| | | | | US | 2023257795 | A1 | 17-08-2023 |
| | | | | WO | 2022008802 | A1 | 13-01-2022 |
| WO 2015069872 | A2 | | 14-05-2015 | US | 2016252515 | A1 | 01-09-2016 |
| | | | | WO | 2015069872 | A2 | 14-05-2015 |
| US 5444053 | A | | 22-08-1995 | AT | E162076 | T1 | 15-01-1998 |
| | | | | AU | 664768 | B2 | 30-11-1995 |
| | | | | CA | 2105912 | A1 | 31-03-1994 |
| | | | | CN | 1089474 | A | 20-07-1994 |
| | | | | DE | 4232899 | A1 | 31-03-1994 |
| | | | | DK | 0590321 | T3 | 14-09-1998 |
| | | | | EP | 0590321 | A1 | 06-04-1994 |
| | | | | ES | 2111104 | T3 | 01-03-1998 |
| | | | | GR | 3026120 | T3 | 29-05-1998 |
| | | | | IL | 106785 | A | 30-09-1997 |
| | | | | JP | 3384847 | B2 | 10-03-2003 |
| | | | | JP | H06192106 | A | 12-07-1994 |
| | | | | NO | 303482 | B1 | 20-07-1998 |
| | | | | TW | 231268 | B | 01-10-1994 |
| | | | | US | 5444053 | A | 22-08-1995 |
| | | | | ZA | 936327 | B | 22-03-1994 |
| WO 2010121010 | A2 | | 21-10-2010 | US | 2012232130 | A1 | 13-09-2012 |
| | | | | WO | 2010121010 | A2 | 21-10-2010 |
| WO 0208405 | A1 | | 31-01-2002 | AU | 7281201 | A | 05-02-2002 |
| | | | | KR | 20020008424 | A | 31-01-2002 |
| | | | | WO | 0208405 | A1 | 31-01-2002 |
| WO 2007035496 | A1 | | 29-03-2007 | CA | 2622523 | A1 | 29-03-2007 |
| | | | | CY | 1123740 | T1 | 24-03-2022 |
| | | | | DK | 1933821 | T3 | 26-10-2020 |
| | | | | EA | 200800819 | A1 | 29-08-2008 |
| | | | | EP | 1933821 | A1 | 25-06-2008 |
| | | | | ES | 2823728 | T3 | 10-05-2021 |
| | | | | HU | E052377 | T2 | 28-04-2021 |
| | | | | JP | 5999816 | B2 | 28-09-2016 |
| | | | | JP | 2009508867 | A | 05-03-2009 |
| | | | | JP | 2013155194 | A | 15-08-2013 |
| | | | | JP | 2015078224 | A | 23-04-2015 |
| | | | | JP | 2016040311 | A | 24-03-2016 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 24 38 2547

This annex lists the patent family members relating to the patent documents cited in the above-mentioned European search report.
The members are as contained in the European Patent Office EDP file on
The European Patent Office is in no way liable for these particulars which are merely given for the purpose of information.

08-11-2024

| Patent document cited in search report | Publication date | Patent family member(s) | | Publication date |
|---|---|---|---|---|
| | | JP | 2017193570 A | 26-10-2017 |
| | | LT | 1933821 T | 10-11-2020 |
| | | PL | 1933821 T3 | 11-01-2021 |
| | | PT | 1933821 T | 15-10-2020 |
| | | SI | 1933821 T1 | 30-11-2020 |
| | | US | 2007072943 A1 | 29-03-2007 |
| | | WO | 2007035496 A1 | 29-03-2007 |

EPO FORM P0459

For more details about this annex : see Official Journal of the European Patent Office, No. 12/82

page 2 of 2

EP 4 653 001 A1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **DIMAURO S** ; **SCHON EA** ; **CARELLI V** ; **HIRANO M**. The clinical maze of mitochondrial neurology. *Nat Rev Neurol.*, 2013, vol. 9 (8), 429-444

- **GORMAN GS** ; **CHINNERY PF** ; **DIMAURO S et al.** Mitochondrial diseases.. *Nat Rev Dis Primers. 2016*, 20 October 2016, vol. 2, 16080